# EUROPÄISCHE PATENTSCHRIFT

(11) **EP 1 727 798 B1**
(45) Veröffentlichungstag und Bekanntmachung des Hinweises auf die Patenterteilung: **29.10.2008**
(21) Anmeldenummer: 05715810.7
(22) Anmeldetag: 05.03.2005
(51) Int. Cl.: C07D 209/34, A61K 31/404

(54) **NEUE ARYL-HALTIGE 5-ACYLINDOLINONE, DEREN HERSTELLUNG UND DEREN VERWENDUNG ALS ARZNEIMITTEL**
NOVEL ARYL-CONTAINING 5-ACYLINDOLINONES, THE PRODUCTION THEREOF AND THEIR USE AS MEDICAMENTS
NOUVELLES 5-ACYLINDOLINONES CONTENANT ARYLE, LEUR PRODUCTION ET LEUR UTILISATION EN TANT QUE MEDICAMENT

(30) Priorität: 12.03.2004 DE 102004012069
(43) Veröffentlichungstag der Anmeldung: 06.12.2006
(73) Patentinhaber: Boehringer Ingelheim International GmbH, 55216 Ingelheim am Rhein (DE); Boehringer Ingelheim Pharma GmbH & Co. KG, 55216 Ingelheim (DE)
(72) Erfinder: HECKEL, Armin, 88400 Biberach (DE); ROTH, Gerald, Jürgen, 88400 Biberach (DE); KLEY, Jörg, 88441 Mittelbiberach (DE); HOERER, Stefan, 88416 Ochsenhausen (DE); UPHUES, Ingo, 88444 Ummendorf (DE)
(74) Vertreter: Hammann, Heinz
(86) Internationale Anmeldenummer: PCT/EP2005/002405
(87) Internationale Veröffentlichungsnummer: WO 2005/087726

(56) Entgegenhaltungen:
- WO-A-00/56710
- WO-A-02/50079
- WO-A-03/027109
- US-B1- 6 350 747
- US-B1- 6 369 086
- BRAMSON H N ET AL: "Oxindole-Based Inhibitors of Cyclin-Dependent Kinase 2 (CDK2): Design, Synthesis, Enzymatic Activities, and X-ray Crystallographic Analysis" JOURNAL OF MEDICINAL CHEMISTRY, AMERICAN CHEMICAL SOCIETY. WASHINGTON, US, Bd. 44, 2001, Seiten 4339-4358, XP002274118 ISSN: 0022-2623

## Beschreibung

Gegenstand der vorliegenden Erfindung sind neue aryl-haltige 5-Acylindolinone der allgemeinen Formel 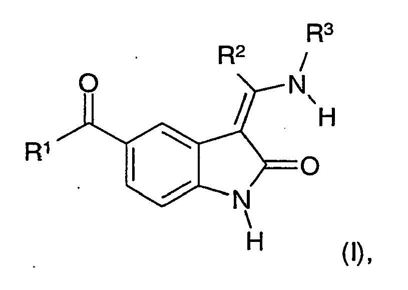 deren Tautomere, deren Enantiomere, deren Diastereomere, deren Gemische und deren Salze, insbesondere deren physiologisch verträgliche Salze mit anorganischen oder organischen Säuren oder Basen, welche wertvolle pharmakologische Eigenschaften aufweisen, beispielsweise eine Hemmwirkung auf Proteinkinasen, insbesondere eine Hemmwirkung auf die Aktivität der Glykogen-Synthase-Kinase (GSK-3) deren Herstellung, deren Verwendung zur Prävention oder Behandlung von Krankheiten oder Zuständen, die in Zusammenhang mit einer veränderten GSK-3 Aktivität stehen insbesondere von Diabetes mellitus Typ I und Typ II, Diabetes assoziierte Störungen wie diabetische Neuropathie, degenerativer neurologischer Erkrankungen wie Alzheimers Erkrankung, Schlaganfall, neurotraumatische Verletzungen, bipolare Störungen, die eine Verbindung der allgemeinen Formel (I) oder ein physiologisch verträgliches Salz davon enthaltenden Arzneimittel sowie Verfahren zu deren Herstellung. Siehe z.B. W02/500 79 als nächsten Stand der Technik.

In der obigen Formel I bedeuten
R¹ eine lineare oder verzweigte C₁₋₅-Alkylgruppe, in der die Wasserstoffatome ganz oder teilweise durch Fluoratome ersetzt sein können, oder
eine gegebenenfalls durch ein Fluor-, Chlor- oder Bromatom substiuierte Arylgruppe,
   wobei unter einer Arylgruppe eine Phenyl- oder Naphthylgruppe zu verstehen ist,
R² eine C₁₋₇-Alkyl- oder C₃₋₇-Cycloalkylgruppe,
eine gegebenenfalls durch ein oder zwei Fluor-, Chlor-, Brom- oder lodatome oder ein oder zwei Nitro-, Cyano-, Amino-, C₁₋₃-Alkyl- oder C₁₋₃-Alkoxygruppen substituierte 5- oder 6-gliedrige Heteroarylgruppe mit ein bis drei Heteroatomen ausgewählt aus der Gruppe N, S und O, wobei sowohl die Heteroatome als auch die Substituenten gleich oder verschieden sein können,
eine Phenylgruppe, in der zwei benachbarte Kohlenstoffatome über eine Methylendioxy-, Ethylendioxy- oder Difluormethylendioxygruppe miteinander verknüpft sind,
eine Phenylgruppe, an die ein weiterer Phenylring oder ein 5- oder 6-gliedriger heteroaromatischer Ring mit ein bis drei Heteroatomen ausgewählt aus der Gruppe N, S und O, wobei die Heteroatome gleich oder verschieden sein können, anneliert ist, und wobei der Bicyclus durch ein oder zwei Fluor-, Chlor-, Brom- oder lodatome oder ein oder zwei Nitro-, Cyano-, Amino-, C₁₋₃-Alkyl- oder C₁₋₃-Alkoxygruppen substituiert sein kann und die Substituenten gleich oder verschieden sein können, oder
eine Phenylgruppe, die durch ein bis drei Fluor-, Chlor-, Brom- oder lodatome oder durch eine bis drei C₁₋₃-Alkyl-, Nitro-, Cyano-, Amino-, Di-(C₁₋₃-alkyl)-amino-, C₁₋₃-Alkyl-carbonylamino-, Phenylcarbonylamino-, C₁₋₃-Alkylsulfonylamino-, Arylsulfonylamino-, Trifluormethyl-, C₁₋₃ Alkylsulfonyl-, Carboxy-, C₁₋₃-Alkoxy-, Di-(C₁₋₃-alkyl)-amino-C₁₋₃-alkyloxy-, C₁₋₃-Alkoxy-carbonyl-, C₁₋₃-Alkylaminocarbonyl-, Hydroxy-carbonyl-C₁₋₃-alkyl-aminocarbonyl-, C₁₋₃-Alkoxycarbonyl-C₁₋₃-alkyl-aminocarbonyl-, Di-(C₁₋₃-alkyl)-amino-C₁₋₃-alkylaminocarbonyl-, Di-(C₁₋₃-alkyl)-amino-carbonyl-C₁₋₃-alkoxy-, C₁₋₃-Alkyl-amino-carbonyl-C₁₋₃-alkoxy-, Carboxy-C₁₋₃-alkoxy-, C₁₋₃-Alkyloxy-carbonyl-C₁₋₃-alkoxy-, Carboxy-C₁₋₃-alkyl-, C₁₋₃-Alkoxy-carbonyl-C₁₋₃-alkyl-, C₁₋₃-Alkoxy-carbonylamino-C₁₋₃-alkyl-, Amino-C₁₋₃-alkyl-, Di-(C₁₋₃-alkyl)-amino-C₁₋₃-alkyl-, C₁₋₃-Alkyl-carbonylamino-C₁₋₃-alkyl-, Phthalimido-, Pyrrolyl- oder Mono- oder Di-(C₁₋₃-alkyl)-pyrrolylgruppen substituiert sein kann, wobei die Substituenten gleich oder verschieden sind, und
R³ eine Phenyl-, Napthyl- oder wie oben definierte Hetroarylgruppe, die
   durch ein Fluor-, Chlor-, Brom- oder Jodatom,
   durch eine Cyano-, Hydroxy-, Carboxy-, C₁₋₃-Alkoxy-, C₁₋₃-Alkoxycarbonyl-oder Di-(C₁₋₃-alkyl)-amino-C₁₋₃-alkoxygruppe,
   durch eine C₁₋₃-Alkylgruppe, die durch eine Hydroxycarbonyl-, C₁₋₃-Alkoxycarbonyl- oder Heteroaryl)gruppe substituiert sein kann,
   durch eine C₁₋₃-Alkylgruppe, die durch eine 3- bis 7-gliedrige Cyclcoalkyleniminogruppe substituiert ist, wobei an die Cycloalkyleniminogruppe über zwei benachbarte Kohlenstoffatome ein Benzolring ankondensiert sein kann,
   durch eine Amino-C₁₋₃-alkylgruppe, die am Stickstoffatom durch eine oder zwei C₁₋₃-Alkyl-, Phenyl-C₁₋₃-alkyl- oder C₁₋₄-Alkoxy-carbonylgruppen substituiert sein kann, wobei die Substituenten gleich oder verschieden sind,
   durch eine C₁₋₃-Alkyl-carbonyl-aminogruppe, die am Stickstoffatom durch eine C₁₋₃-Alkylgruppe oder eine endständig durch eine Di-(C₁₋₃-alkyl)-aminogruppe substituierte C₂₋₃-Alkylgruppe und im Alkylteil durch eine Di-(C₁₋₃-alkyl)-amino-, Piperazinyl- oder 4-(C₁₋₃-Alkyl)-piperazin-1-ylgruppe substituiert sein kann,
   durch eine im Alkylteil endständig durch eine Di-(C₁₋₃-alkyl)-aminogruppe substituierte C₂₋₃-Alkyl-aminocarbonylgruppe, die zusätzlich am Stickstoffatom durch eine C₁₋₃-Alkylgruppe substituiert sein kann,
   oder durch eine Heteroarylgruppe
mono-, di- oder trisubstituiert sein kann, wobei die Substituenten gleich oder verschieden sein können, bedeuten,
   wobei die vorstehend erwähnten Alkylgruppen geradkettig oder verzweigt sein können,
deren Tautomere, Enantiomere, Diastereomere, deren Gemische und deren Salze.

Soweit nichts anderes erwähnt wurde, ist unter einer 5-gliedrigen Heteroarylgruppe bevorzugterweise eine Furanyl-, Thiophenyl-, Pyrrolyl-, Pyrazolyl-, Thiazolyl-, Imidazolyl-, Oxazolyl-, Triazolyl- oder Thiadiazolylgruppe, und unter einer 6-gliedrigen Heteroarylgruppe eine Pyridinyl-, Pyrimidinyl-, Pyridazinyl- oder Pyrazinylgruppe zu verstehen.

Unter einer Arylgruppe, ist, soweit nicht anderes erwähnt wird, eine Phenyl- oder Naphthylgruppe zu verstehen; bevorzugt ist die Phenylgruppe.

Bevorzugt sind diejenigen Verbindungen der allgemeinen Formel I, in denen
R² und R³ wie vorstehend erwähnt definiert sind und
R¹ eine Methyl-, Ethyl-, n-Propyl-, Isopropyl-, n-Pentyl-, Trifluormethyl- oder Phenylgruppe bedeutet,
deren Tautomere, Enantiomere, Diastereomere, deren Gemische und deren Salze.

Besonders bevorzugt sind diejenigen Verbindungen der allgemeinen Formel I, in denen
R¹ eine Methyl-, Ethyl-, n-Propyl-, Isopropyl-, n-Pentyl- oder Phenylgruppe,
R² C₁₋₇-Alkylgruppe,
eine Phenylgruppe, in der zwei benachbarte Kohlenstoffatome über eine Methylendioxy-, Ethylendioxy- oder Difluormethylendioxygruppe miteinander verknüpft sind, oder
eine Phenylgruppe, die durch ein oder zwei Fluor-, Chlor-, Brom- oder Iodatome oder durch eine oder zwei C₁₋₃-Alkyl-, Nitro-, Cyano-, Amino-, C₁₋₃-Alkylcarbonylamino-, Phenylcarbonylamino-, C₁₋₃-Alkylsulfonylamino-, Trifluormethyl-, Carboxy-, C₁₋₃-Alkoxy-, Di-(C₁₋₃-alkyl)-amino-C₁₋₃-alkyloxy-, C₁₋₃-Alkoxy-carbonyl-, C₁₋₃-Alkylamino-carbonyl-, Hydroxycarbonyl-C₁₋₃-alkyl-aminocarbonyl-, C₁₋₃-Alkoxycarbonyl-C₁₋₃-alkyl-aminocarbonyl-, Di-(C₁₋₃-alkyl)-amino-C₁₋₃-alkylaminocarbonyl-, Carboxy-C₁₋₃-alkyl-, C₁₋₃-Alkoxy-carbonyl-C₁₋₃-alkyl-, Amino-C₁₋₃-alkyl- oder C₁₋₃-Alkyl-carbonylamino-C₁₋₃-alkylgruppen substituiert sein kann, wobei die Substituenten gleich oder verschieden sind, und
R³ eine Phenylgruppe, die
   durch ein Fluor-, Chlor- oder Bromatom,
   durch eine Cyano-, Hydroxy-, Carboxy-, C₁₋₃-Alkoxy-, C₁₋₃-Alkoxycarbonyl-oder Di-(C₁₋₃-alkyl)-amino-C₁₋₃-alkoxygruppe,
   durch eine C₁₋₃-Alkylgruppe, die durch eine Hydroxycarbonyl-, C₁₋₃-Alkoxycarbonyl- oder Imidazolylgruppe substituiert sein kann,
   durch eine C₁₋₃-Alkylgruppe, die durch eine 3- bis 7-gliedrige Cyclcoalkyleniminogruppe substituiert ist, wobei an die Cycloalkyleniminogruppe über zwei benachbarte Kohlenstoffatome ein Benzolring ankondensiert sein kann,
   durch eine Amino-C₁₋₃-alkylgruppe, die am Stickstoffatom durch eine oder zwei C₁₋₃-Alkyl-, Benzyl- oder C₁₋₄-Alkoxy-carbonylgruppen substituiert sein kann, wobei die Substituenten gleich oder verschieden sind,
   durch eine C₁₋₃-Alkyl-carbonyl-aminogruppe, die am Stickstoffatom durch eine C₁₋₃-Alkylgruppe oder eine endständig durch eine Di-(C₁₋₃-alkyl)-aminogruppe substituierte C₂₋₃-Alkylgruppe und im Alkylteil durch eine Di-(C₁₋₃-alkyl)-amino-, Piperazinyl- oder 4-(C₁₋₃-Alkyl)-piperazin-1-ylgruppe substituiert sein kann,
   durch eine im Alkylteil endständig durch eine Di-(C₁₋₃-alkyl)-aminogruppe substituierte C₂₋₃-Alkyl-aminocarbonylgruppe, die zusätzlich am Stickstoffatom durch eine C₁₋₃-Alkylgruppe substituiert sein kann,
   oder durch eine Imidazolylgruppe
mono- oder disubstituiert sein kann, wobei die Substituenten gleich oder verschieden sein können, bedeuten,
insbesondere jedoch diejenigen Verbindungen, in denen
R¹ eine Methylgruppe,
R² eine Ethyl-, Propyl-, Butyl- oder Pentylgruppe,
eine Phenylgruppe, in der zwei benachbarte Kohlenstoffatome über eine Methylendioxy-, Ethylendioxy- oder Difluormethylendioxygruppe miteinander verknüpft sind, oder
eine Phenylgruppe, die durch ein oder zwei Fluor-, Chlor-, Bromatome oder durch eine oder zwei C₁₋₃-Alkyl-, Cyano-, C₁₋₃-Alkoxy-, Carboxy-C₁₋₃-alkyl-, C₁₋₃-Alkoxycarbonyl-C₁₋₃-alkyl-, Amino-C₁₋₃-alkyl- oder C₁₋₃-Alkyl-carbonylamino-C₁₋₃-alkylgruppen substituiert sein kann, wobei die Substituenten gleich oder verschieden sind, und
R³ eine Phenylgruppe, die
   durch ein Fluor-, Chlor- oder Bromatom,
   durch eine Cyano-, Carboxy-, C₁₋₃-Alkoxy- oder C₁₋₃-Alkoxycarbonylgruppe,
   durch eine C₁₋₃-Alkylgruppe, die durch eine Hydroxycarbonyl- oder C₁₋₃-Alkoxy-carbonylgruppe substituiert sein kann,
   durch eine C₁₋₃-Alkylgruppe, die durch eine 3- bis 7-gliedrige Cyclcoalkyleniminogruppe substituiert ist,
   durch eine Amino-C₁₋₃-alkylgruppe, die am Stickstoffatom durch eine oder zwei C₁₋₃-Alkyl- oder C₁₋₄-Alkoxy-carbonylgruppen substituiert sein kann, wobei die Substituenten gleich oder verschieden sind,
   durch eine C₁₋₃-Alkyl-carbonyl-aminogruppe, die am Stickstoffatom durch eine C₁₋₃-Alkylgruppe oder eine endständig durch eine Di-(C₁₋₃-alkyl)-aminogruppe substituierte C₂₋₃-Alkylgruppe und im Alkylteil durch eine Di-(C₁₋₃-alkyl)-amino-oder 4-(Methyl)-piperazin-1-ylgruppe substituiert sein kann,
   oder durch eine im Alkylteil endständig durch eine Di-(C₁₋₃-alkyl)-aminogruppe substituierte C₂₋₃-Alkyl-aminocarbonylgruppe, die zusätzlich am Stickstoffatom durch eine C₁₋₃-Alkylgruppe substituiert sein kann,
monosubstituiert oder durch eine Hydroxy- und eine Di-(C₁₋₃-alkyl)-amino-C₁₋₃-alkylgruppe disubstituiert sein kann, wobei die Substituenten gleich oder verschieden sein können, bedeuten,
   wobei die vorstehend erwähnten Alkylgruppen geradkettig oder verzweigt sein können,
deren Tautomere, Enantiomere, Diastereomere, deren Gemische und deren Salze.

Ganz besonders bevorzugt sind diejenigen Verbindungen der allgemeinen Formel I, in denen
R¹ eine Methylgruppe,
R² eine Phenylgruppe, in der zwei benachbarte Kohlenstoffatome über eine Methylendioxy- oder Ethylendioxygruppe miteinander verknüpft sind, oder
eine Phenylgruppe, die durch ein oder zwei Methoxygruppen substituiert sein kann, und
R³ eine eine Phenylgruppe, die
   durch eine Cyanogruppe oder
   durch eine Amino-C₁₋₃-alkylgruppe, die am Stickstoffatom durch eine oder zwei C₁₋₃-Alkylgruppen substituiert sein kann, wobei die Substituenten gleich oder verschieden sein können,
substituiert ist, bedeuten,
   wobei die vorstehend erwähnten Alkylgruppen geradkettig oder verzweigt sein können,
deren Tautomere, Enantiomere, Diastereomere, deren Gemische und deren Salze;
insbesondere sind folgende Verbindungen der allgemeinen Formel I zu nennen:
   (a) 5-Acetyl-3-{[4-(diethylaminomethyl)-phenylamino]-phenyl-methyliden}-2-indolinon 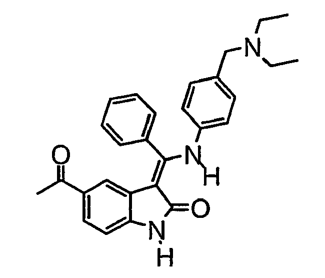
   (b) 5-Acetyl-3-{[4-(dimethylamino-methyl)-phenylamino]-(2,3-dihydro-benzo[1,4]-dioxin-6-yl)-methyliden}-2-indolinon 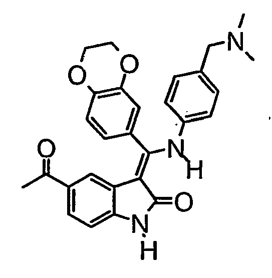
   (c) 5-Acetyl-3-{[4-(dimethylaminomethyl)-phenylamino]-(3-methoxy-phenyl)-methyliden}-2-indolinon 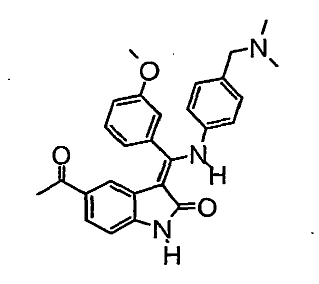
   (d) 5-Acetyl-3-{[4-(dimethylaminomethyl)-phenylamino]-(3,5-dimethoxy-phenyl)-methyliden}-2-indolinon 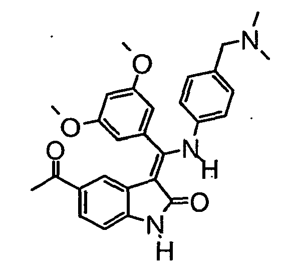
   (e) 5-Acetyl-3-[(4-cyano-phenylamino)-(2,3-dihydro-benzo[1,4]dioxin-6-yl)-methyliden]-2-indolinon 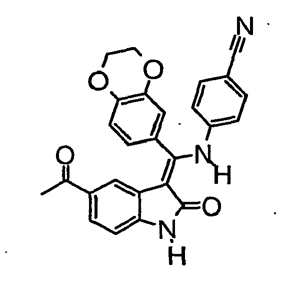
   (f) 5-Acetyl-3-{[4-(ethylaminomethyl)-phenylamino]-phenyl-methyliden}-2-indolinon 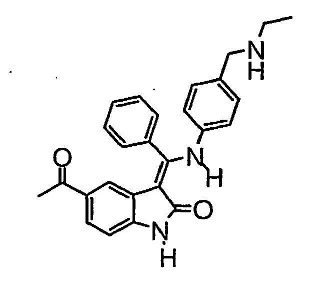
   (g) 5-Acetyl-3-[1-(4-(dimethylaminomethyl)-phenylamino)-butyliden)-2-indolinon 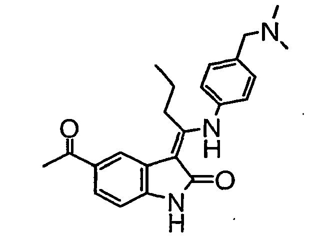
   (h) 5-Acetyl-3-[1-(4-(dimethylaminomethyl)-phenylamino)-propyliden)-2-indolinon 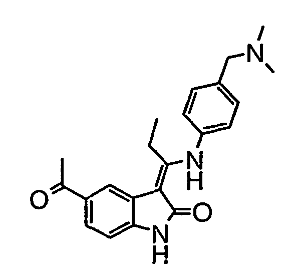
   sowie deren Tautomere, Enantiomere, Diastereomere, deren Gemische und deren Salze.

Erfindungsgemäß erhält man die Verbindungen der allgemeinen Formel I nach an sich bekannten Verfahren, beispielsweise nach folgenden Verfahren:
a) Umsetzung einer Verbindung der allgemeinen Formel 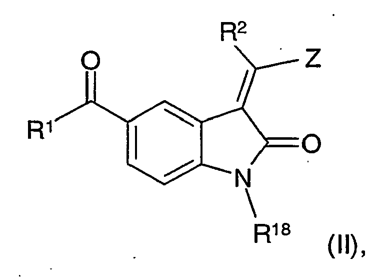 in der R¹ und R² wie eingangs erwähnt definiert sind,
   R¹⁸ ein Wasserstoffatom oder eine Schutzgruppe für das Stickstoffatom der Lactamgruppe und
   Z eine Austrittsgruppe wie etwa ein Halogenatom, eine Hydroxy-, Alkoxy-,Alkylsulfonyl-, Alkyl-arylsulfonyl-, Trialkylsilyloxy- oder Aryl-alkoxygruppe, z.B. ein Chlor-oder Bromatom, eine Methoxy-, Ethoxy-, Methansulfonyl-, Toluolsulfonyl-, Trimethylsilyloxy- oder Benzyloxygruppe, bedeuten,
   mit einem "Amin der allgemeinen Formel

   R³-NH₂ (III)

   ,
   in der R³ wie eingangs erwähnt definiert ist,
   wobei eventuell in den Resten R² und/oder R³ enthaltene Hydroxy-, Amino- oder Iminogruppen vorübergehend durch geeignete Schutzgruppen geschützt werden können;
   und erforderlichenfalls anschließende Abspaltung einer verwendeten Schutzgruppe für das Stickstoffatom der Lactam- oder Iminogruppe.
   Als Schutzgruppe für das Stickstoffatom der Lactamgruppe kommt beispielsweise eine Acetyl-, Benzoyl-, Ethoxycarbonyl-, tert.Butyloxycarbonyl- oder Benzyloxycarbonylgruppe und
   Die Umsetzung wird zweckmäßigerweise in einem Lösungsmittel wie Dimethylformamid, Toluol, Acetonitril, Tetrahydrofuran, Dimethylsulfoxid, Methylenchlorid oder deren Gemischen gegebenenfalls in Gegenwart einer inerten Base wie Triethylamin, N-Ethyl-diisopropylamin oder Natriumhydrogencarbonat bei Temperaturen zwischen 20 und 175°C durchgeführt, wobei eine verwendete Schutzgruppe gleichzeitig abgespalten werden kann.
   Bedeutet Z in einer Verbindung der allgemeinen Formel II ein Halogenatom, dann wird die Umsetzung vorzugsweise in Gegenwart einer inerten Base bei Temperaturen zwischen 20 und 120°C, durchgeführt.
   Bedeutet Z in einer Verbindung der allgemeinen Formel II eine Hydroxy-, Alkoxy-oder Arylalkoxygruppe, dann wird die Umsetzung vorzugsweise bei Temperaturen zwischen 20 und 200°C, durchgeführt.
   Die gegebenenfalls erforderliche anschließende Abspaltung einer verwendeten Schutzgruppe wird zweckmäßigerweise entweder hydrolytisch in einem wäßrigen oder alkoholischen Lösungsmittel, z.B. in Methanol/Wasser, Ethanol/Wasser, Isopropanol/Wasser, Tetrahydrofuran/Wasser, Dioxan/Wasser, Dimethylformamid/Wasser, Methanol oder Ethanol in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C,
   oder vorteilhafterweise durch Umamidierung mit einer organischen Base wie Ammoniak, Butylamin, Dimethylamin oder Piperidin in einem Lösungsmittel wie Methanol, Ethanol, Dimethylformamid und deren Gemischen oder in einem Überschuß des eingesetzten Amins bei Temperaturen zwischen 0 und 100°C, vorzugsweise bei Temperaturen zwischen 10 und 50°C, durchgeführt.
b) zur Herstellung einer Verbindung der Formel I, die eine Aminocarbonylgruppe enthält: Umsetzung einer Verbindung, die eine Carboxygruppe enthält, mit dem entsprechenden Amin zur entsprechenden Aminocarbonylverbindung;
c) zur Herstellung einer Verbindung der Formel I, die eine Carbonylaminogruppe enthält: Umsetzung einer Verbindung, die eine Aminogruppe enthält, mit dem entsprechenden Säurechlorid zur entsprechenden Carbonylaminoverbindung;
d) zur Herstellung einer Verbindung der Formel I, die eine Aminomethylgruppe enthält: Hydrierung einer Verbindung, die eine Cyanogruppe enthält, zu dem entsprechenden Aminomethylderivat;
e) zur Herstellung einer Verbindung der Formel I, die eine Aminogruppe enthält: Reduktion einer Verbindung, die eine Nitrogruppe enthält.

Anschließend können gegegebenenfalls während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren oder Basen, übergeführt werden.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsstoffe verwendeten Verbindungen der allgemeinen Formeln II bis III sind entweder literaturbekannt oder man erhält diese nach an sich literaturbekannten Verfahren (siehe Beispiele I bis VII).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym GSK-3.

Glykogen Synthase Kinase-3 (GSK-3) ist eine Serin/Threonin-Kinase die in zwei Isoformen, GSK-3α und GSK-3β, existiert. GSK-3 phosphoryliert und inaktiviert die Glykogensynthase, ein Schlüsselenzym der insulinabhängigen Regulation der Glykogensynthese (Embi et al., Eur. J. Biochem. 107, 519-527, (1980)), aber in vitro auch eine Vielzahl weiterer regulatorischer Proteine. Zu diesen Proteinen gehören das Mikrotubulus assoziierte Protein Tau, Elongation initiation factor 2b (eIF2b), β-Catenin, Axin, ATP-Citratlyase, Heat-shock-factor 1, c-Jun, c-myc, c-myb, CREB und CEBPα. Diese verschiedenen Substrate implizieren eine Rolle für GSK-3 in vielen Bereichen des zellulären Metabolismus, der Proliferation, der Differenzierung und der Entwicklung.

Typ 2 Diabetes wird durch eine Insulinresistenz in verschiedenen Geweben wie Skelettmuskel, Leber und Fettgewebe und durch eine veränderte Insulinsekretion der Bauchspeicheldrüse gekennzeichnet. Die Speicherung von Glykogen in Leber und Muskel ist von großer Bedeutung für die Aufrechterhaltung des Glukosegleichgewichts. Im Typ 2 Diabetes ist die Aktivität der Glykogensynthase vermindert und somit die Rate der Glykogensynthese reduziert. Außerdem konnte gezeigt werden, dass GSK-3 im Typ 2 diabetischen Muskel verstärkt exprimiert wird und dass somit eine vermehrte GSK-3 Aktivität mit einer verminderten Glykogensyntheserate einhergeht (Nikoulina et al., Diabetes 49, 263-271, (2000)). Eine Hemmung der GSK-3 Aktivität stimuliert die Glykogensynthase, verstärkt somit die Glykogensynthese und führt letztlich zu einer Reduktion der Glukosespiegel. Eine GSK-3 Hemmung ist daher von therapeutischer Relevanz für die Behandlung von Typ 1 und Typ 2 Diabetes sowie von diabetischer Neuropathie.

Die Alzheimersche Erkrankung ist dadurch gekennzeichnet, dass das Mikrotubulus assoziierte Protein Tau übermäßig stark phosphoryliert vorliegt (Cohen & Frame, Nature Reviews: Molecular Cell Biology, 2, 1-8, (2001)). GSK-3 phosphoryliert viele dieser Phosphorylierungsstellen von Tau in vitro, wodurch die Bindung an Mikrotubuli verhindert wird. Hierdurch steht Tau für eine vermehrte Filamentassemblierung zur Verfügung, die in der Alzheimerschen Erkrankung und anderen neurologischen Erkrankungen der neuronalen Degeneration zugrunde liegt. Es konnte gezeigt werden dass GSK-3 Inhibitoren, wie Insulin oder Lithium eine partielle Dephosphorylierung von Tau in neuronalen Zellen bewirken (Cross et al., J. Neurochem. 77, 94-102 (2001)). Eine GSK-3 Hemmung kann daher von therapeutischer Relevanz für die Behandlung degenerativer neurologischer Erkrankungen wie der Alzheimerschen Erkrankung sein.

Inhibitoren der GSK-3 Aktivität können somit therapeutisch und /oder präventiv für eine Reihe von Erkrankungen nützlich sein, bei denen eine GSK-3 Hemmung hilfreich ist, wie bei Diabetes und Diabetes assoziierten Erkrankungen, chronisch neurodegenerativen Erkrankungen und Demenzen, wie der Alzheimerschen Erkrankung, beim Parkinson Syndrom, Pick's-Erkrankungen, Demenz bei subkortikaler arteriosklerotischer Enzephalopathie (SAE), Chorea Huntington, multiple Sklerose, infektiöse Erkrankungen (Meningoenzephalitiden, Lues, Hirnabszeß, Creutzfeldt-Jakob-Krankheit, AIDS), Demenzkomplex mit Lewy-Körperchen, neurotraumatischen Erkrankungen wie akuter Schlaganfall, Schizophrenie, manischer Depression, Hirnblutung, Haarausfall, Adipositas, atherosklerotische kardiovaskuläre Erkrankungen, Bluthochdruck, PCO-Syndrom, Metabolisches Syndrom, Ischämie, Krebs, Leukopenie, Down Syndrom, Entzündungen, Immundefizienz.

Eine neue Studie (Sato, N. et al., Nature Medicine 10, 55-63 (2004)) zeigt, dass GSK-3 Inhibitoren die Pluripotenz von Stammzellen erhalten können, was neue Anwendungsmöglichkeiten im Rahmen regenerativer Therapien durch Stammzellen ermöglichen kann.

### Bestimmung der GSK-3 Aktivität

Die Wirkung von Substanzen auf die GSK-3 Aktivität wurde nach folgendem Versuchsprotokoll durchgeführt, das auf der Phosphorylierung eines 26mer Peptids (YRRAAVPPSPSLSRHSSFHQpSEDEEE) aus der Glykogensynthase beruht, dessen Sequenz die Phosphorylierungsstellen für GSK-3 aufweist und dessen Vorphosphorylierung mit (pS) gekennzeichnet ist.

Die Testsubstanz wird in DMSO/Wasser gelöst. GSK3β (Universität Dundee, UK) gelöst in 10 mM MOPS (Morpholinopropansulfonsäure), 0.05 mM EDTA, 0.005% Brij, 2.5 % Glycerin, 0.05 % Mercaptoethanol, pH 7.0, wird mit 10 µM [³³P]-ATP, 0.25 µM 26mer Peptid versetzt und mit der gelösten Substanz in 50 mM Tris, 10 mM MgCl₂, 0.1 % Mercaptoethanol, pH 7.5, bei Raumtemperatur inkubiert. Die Reaktion wurde durch Zugabe von 75 mM Phosphorsäure gestoppt. Der Reaktionsansatz wurde auf Phosphocellulose Filterplatten (Millipore) transferiert und bis zur trockne gefiltert und zweimal mit 75 mM Phosphorsäure gewaschen. Die Phosphorylierung wurde durch die Messung der Radioaktivität auf dem Filter in einem Szintillationscounter (Topcount, Packard) bestimmt. Die Fähigkeit einer Substanz zur GSK-3 Hemmung wird durch den Vergleich des Signals eines Reaktionsansatzes mit verschiedenen Substanzkonzentrationen mit dem Signal des Reaktionsansatzes ohne Substanz bestimmt. Die IC₅₀-Werte werden durch nicht lineare Regressionsanalyse mit Hilfe der GraphPad Prism Software berechnet.
Typische IC₅₀-Werte für die untersuchten Substanzen lagen zwischen 0.0001 µM und 1 µM.

### Bestimmung der Glykogensynthese

Dieser Test dient dazu, die Wirkung von Testsubstanzen auf die Glykogensynthese in Zellen zu untersuchen.
C3A hepatoma Zellen (ATCC) werden mit einer Dichte von 100000 Zellen/ml in 96well Platten ausgesät und als Monolayer im Medium bis zur Konfluenz kultiviert. Das Medium wird entfernt und die Zellen werden mehrmals mit PBS gewaschen und anschließend in KRBH-Puffer (134 mM NaCl, 3.5 mM KCI, 1.2 mM KH₂PO₄, 0.5 mM MgSO₄, 1.5 mM CaCl₂, 5 mM NaHCO₃, 10 mM HEPES, pH 7.4) mit 0.1 % BSA und 0.5 mM Glukose für 60 min bei 37°C inkubiert. Testsubstanz und 0.2 µCi D-[U¹⁴C]-Glukose (Amersham) werden zugefügt und die Zellen für weitere 60 min unter den gleichen Bedingungen inkubiert. Nach Entfernung des Inkubationspuffers werden die Zellen mehrmals mit kaltem PBS gewaschen und dann 10 min bei 37°C und 10 min bei Raumtemperatur mit 1 M NaOH lysiert. Die Zell-Lysate werden auf Filterplatten transferiert und das Gkykogen wird durch 2 h Inkubation mit kaltem Ethanol (70%) auf Eis präzipitiert. Die Präzipitate werden mehrmals mit Ethanol gewaschen und bis zur trockne filtriert. Das synthetisierte Glykogen wird durch die Messung der Radioaktivität (eingebaute 14C-Glucose) auf den Filterplatten in einem Szintillationscounter (Topcount, Packard) bestimmt.
Die Fähigkeit einer Substanz zur Stimulation der Glykogensynthese wird durch den Vergleich des Signals eines Reaktionsansatzes mit verschiedenen Substanzkonzentrationen mit dem Signal des Reaktionsansatzes ohne Substanz bestimmt.

### Oraler Glukose Toleranztest

7 - 9 Wochen alte gefastete db/db-Mäuse (Janvier, Frankreich) werden gewogen und Blut wird aus der Schwanzspitze entnommen. Dieses Blut dient der erste Glukosemessung auf Grund derer die Tiere randomisiert und in Gruppen eingeteilt werden. Die zu überprüfende Testsubstanz kann entweder oral oder i.p. als Suspension in 0.5 % Natrosol verabreicht werden. 30 Minuten nach Substanzgabe wird den Tieren oral 2 g/kg Glukose in einem Volumen von 0,1 ml/100 g Körpergewicht gelöst in NaCl-Lösung verabreicht. Im Anschluss werden aus Schwanzblut in bestimmten Zeitabständen [30, 60, 120 und 180 Minuten nach oraler Glukosegabe] die Glukosewerte mit einem Glukometer (Ultra OneTouch, Lifescan) bestimmt.
Beispielsweise zeigt Verbindung 1.051 eine deutliche Wirkung im oralen Glukosetoleranztest.

Die erfindungsgemäß hergestellten Verbindungen sind gut verträglich, da beispielsweise nach oraler Gabe von 10 mg/kg der Verbindung des Beispiels 1.051 an Ratten keine Änderungen im Verhalten der Tiere beobachtet werden konnten.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. GI 262570) und -Antagonisten, PPAR-gamma/alpha Modulatoren (z.B. KRP 297), alpha-Glucosidasehemmer (z.B. Acarbose, Voglibose), DPP-IV-Inhibitoren, alpha2-Antagonisten, Insulin und Insulinanaloga, GLP-1 und GLP-1 Analoga (z.B. Exendin-4) oder Amylin. Daneben SGLT2-Inhibitoren wie T-1095, Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptorantagonisten und Inhibitoren der Phosphoenolpyruvatcarboxykinase, der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha agonisten, PPAR-delta-agonisten, ACAT-Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder β₃-Agonisten wie SB-418790 oder AD-9677 ebenso wie Agonisten des 5HT2c Rezeptors.

Daneben ist eine Kombination mit Medikamenten zur Beeinflussung des Bluthochdrucks wie z.B. All Antagonisten oder ACE Inhibitoren, Diuretika, β-Blocker, Ca-Antagonisten und anderen oder Kombinationen daraus geeignet.

Im Allgemeinen können GSK-3-Inhibitoren auf verschienen Wegen dargereicht werden: oral, transdermal, intranasal, parenteral oder unter besonderen Umständen intrarectal. Die bevorzugte Darreichungsform ist die tägliche orale Gabe, die mehrmals am Tag erfolgen kann. GSK-3-Inhibitoren sind über einen weiten Dosisbereich wirksam. So kann die Dosierung beispielsweise zwischen 0.001 und 100 mg/kg liegen.

Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:
Herstellung der Ausgangsverbindungen:

### Beispiel I

| 5-Acetyl-2-indolinon |
|---|
| 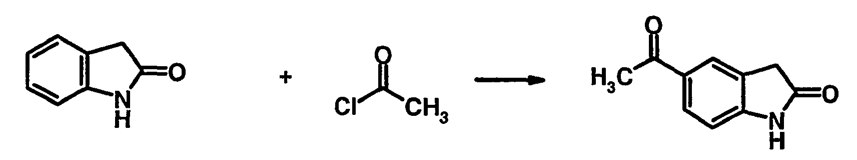 |

171 g (1,28 mol) Aluminiumchlorid werden in 500 ml 1,2-Dichlorethan im Eisbad gekühlt. Dann werden 78 g (1,1 mol) Acetylchlorid zugetropft, so dass die Temperatur 10°C nicht überschreitet. Nach 1 h werden 71,3 g (0,53 mol) 2-Indolinon (1,3-Di-hydro-indol-2-on) in 4 Portionen zugegeben und die Temperatur bei 10-12°C gehalten. Das Reaktionsgemisch lässt man über Nacht langsam auf Raumtemperatur erwärmen. Anschließend wird die Lösung langsam und unter starkem Rühren zu 1 kg Eis gegeben. Der Brei wird mit 1 l Wasser verdünnt und noch 30 min nachgerührt. Dann wird der Niederschlag abgesaugt.
Ausbeute: 80,9 g (86,3 % der Theorie)
R_{f} = 0,36 (Kieselgel, Essigester/Cyclohexan/Methanol 9:9:2)
C₁₀H₉NO₂ (MG = 175,19)
Massenspektrum: m/z = 174 (M-H)⁻

Analog Beispiel I werden folgende Verbindungen hergestellt:
(1) 5-Propionly-2-indolinon
   Hergestellt aus 2-Indolinon und Propionylchlorid
   Ausbeute: 72 % der Theorie
   R_{f} = 0,44 (Kieselgel, Methylenchlorid/Methanol 9:1)
   C₁₁H₁₁NO₂ (MG = 189,22)
   Massenspektrum: m/z = 188 (M-H)⁻
(2) 5-Butyryl-2-indolinon
   Hergestellt aus 2-Indolinon und Buttersäurechlorid (Butyrylchlorid)
   Ausbeute: 68 % der Theorie
   C₁₂H₁₃NO₂ (MG = 203,24)
   Massenspektrum: m/z = 202 (M-H)⁻
(3) 5-Isobutyryl-2-indolinon
   Hergestellt aus 2-Indolinon und Isobutyrylchlorid
   Ausbeute: 13 % der Theorie
   C₁₂H₁₃NO₂ (MG = 203,24)
   Massenspektrum: m/z = 202 (M-H)⁻
(4) 5-Hexanoyl-2-indolinon
   Hergestellt aus 2-Indolinon und Hexansäurechlorid Ausbeute: 88 % der Theorie
   R_{f} = 0,51 (Kieselgel, Essigester/Cyclohexan/Methanol 9:9:2)
   C₁₄H₁₇NO₂ (MG = 231,30)
   Massenspektrum: m/z = 230 (M-H)⁻
(5) 5-Benzoyl-2-indolinon
   Hergestellt aus 2-Indolinon und Benzoesäurechlorid Ausbeute: 80 % der Theorie
   R_{f} = 0,46 (Kieselgel, Methylenchlorid/Methanol 9:1)
   C₁₅H₁₁NO₂ (MG = 237,26)
   Massenspektrum: m/z = 236 (M-H)⁻

### Beispiel II

| 1,5-Diacetyl-2-indolinon |
|---|
| 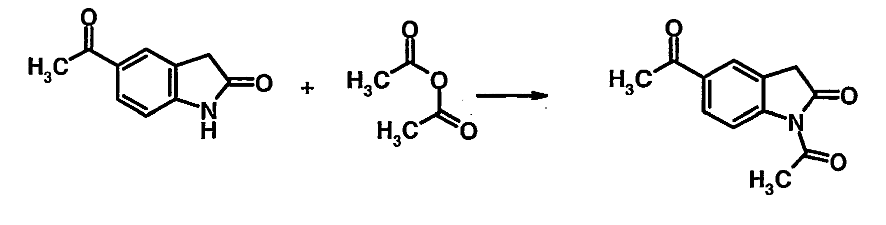 |

48,9 g (0,279 mol) 5-Acetyl-2-indolinon werden in 400 ml Essigsäureanhydrid 2 h in einem Ölbad bei 140 °C gerührt. Dabei löst sich das Ausgangsmaterial auf. Anschließend lässt man das Reaktionsgemisch abkühlen, engt ein, saugt den Niederschlag ab, wäscht ihn mit Ether und trocknet das Produkt.
Ausbeute: 56,0 g (92,4 % der Theorie)
R_{f} = 0,41 (Kieselgel, Methylenchlorid/Methanol 50:1)
C₁₂H₁₁NO₃ (MG = 217,223)
Massenspektrum: m/z = 216 (M-H)⁻

Analog Beispiel II werden folgende Verbindungen hergestellt:
(1) 1-Acetyl-5-propionly-2-indolinon
   Hergestellt aus 5-Propionly-2-indolinon und Essigsäureanhydrid
   Ausbeute: 79 % der Theorie
   R_{f} = 0,68 (Kieselgel, Methylenchlorid/Methanol 9:1)
   C₁₃H₁₃NO₃ (MG = 231,25)
   Massenspektrum: m/z = 232 (M+H)⁺
(2) 1-Acetyl-5-benzoyl-2-indolinon
   Hergestellt aus 5-Benzoyl-2-indolinon und Essigsäureanhydrid
   Ausbeute: 89 % der Theorie
   R_{f} = 0,60 (Kieselgel, Methylenchlorid/Methanol 30:1)
   C₁₇H₁₃NO₃ (MG = 279,294)
   Massenspektrum: m/z = 278 (M-H)⁻
(3) 1-Acetyl-5-hexanoyl-2-indolinon
   Hergestellt aus 5-Hexanoyl-2-indolinon und Essigsäureanhydrid
   R_{f} = 0,74 (Kieselgel, Methylenchlorid/Methanol 30:1)
   C₁₆H₁₉NO₃ (MG = 273,33)
   Massenspektrum: m/z = 272 (M-H)⁻

### Beispiel III

| 1,5-Diacetyl-3-(ethoxy-phenyl-methyliden)-2-indolinon |
|---|
| 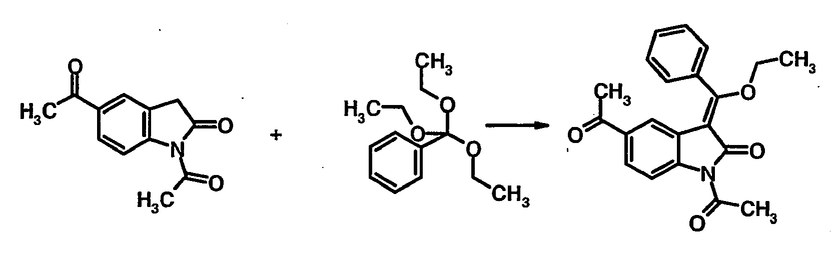 |

32,6 g (150 mmol) 1,5-Diacetyl-2-indolinon werden in 100 ml Orthobenzoesäuretriethylester suspendiert und mit 150 ml Essigsäureanhydrid bei 110 °C über Nacht gerührt. Dann werden nochmals 50 ml Orthobenzoesäuretriethylester zugegeben und weitere 24 h gerührt. Anschließend wird eingeengt und der entstehende Niederschlag abgesaugt, gewaschen und getrocknet.
Ausbeute: 38 g (72,5 % der Theorie)
R_{f} = 0,60 (Kieselgel, Methylenchlorid/Methanol 30:1)
C₂₁H₁₉NO₄ (MG = 349,384)
Massenspektrum: m/z = 350 (M+H)⁺

Analog Beispiel III werden folgende Verbindungen hergestellt:
(1) 1-Acetyl-5-hexanoyl-3-(ethoxy-phenyl-methyliden)-2-indolinon
   Hergestellt aus 1-Acetyl-5-hexanoyl-2-indolinon und Orthobenzoesäuretriethylester
   Ausbeute: 29 % der Theorie
   R_{f} = 0,72 (Kieselgel, Methylenchlorid/Methanol 30:1)
   C₂₅H₂₇NO₄ (MG = 405,491)
   Massenspektrum: m/z = 428 (M+Na)⁺
(2) 1-Acetyl-5-benzoyl-3-(ethoxy-phenyl-methyliden)-2-indolinon
   Hergestellt aus 1-Acetyl-5-benzoyl-2-indolinon und Orthobenzoesäuretriethylester
   Ausbeute: 65 % der Theorie
   R_{f} = 0,72 (Kieselgel, Methylenchlorid/Methanol 30:1)
   C₂₆H₂₁NO₄ (MG = 411,455)
   Massenspektrum: m/z = 412 (M+H)⁺
(3) 1,5-Diacetyl-3-(1-methoxy-propyliden)-2-indolinon
   Hergestellt aus 1,5-Diacetyl-2-indolinon und Orthopropionsäuretrimethylester
   Ausbeute: 80 % der Theorie
   R_{f} = 0,50 (Kieselgel, Methylenchlorid/Methanol 50:1)
   C₁₆H₁₇NO₄ (MG = 287,311)
   Massenspektrum: m/z = 288 (M+H)⁺
(4) 1,5-Diacetyl-3-(1-methoxy-butylidden)-2-indolinon
   Hergestellt aus 1,5-Diacetyl-2-indolinon und Orthobuttersäuretrimethylester
   Ausbeute: 71 % der Theorie
   R_{f} = 0,53 (Kieselgel, Methylenchlorid/Methanol 50:1)
   C₁₇H₁₉NO₄ (MG = 301,337)
   Massenspektrum: m/z = 302 (M+H)⁺
(5) 1,5-Diacetyl-3-(1-methoxy-pentyliden)-2-indolinon
   Hergestellt aus 1,5-Diacetyl-2-indolinon und Orthovaleriansäuretrimethylester
   Ausbeute: 66 % der Theorie
   R_{f} = 0,60 (Kieselgel, Methylenchlorid/Methanol 50:1)
   C₁₈H₂₁NO₄ (MG = 315,364)
   Massenspektrum: m/z = 316 (M+H)⁺
(6) 1,5-Diacetyl-3-(1-methoxy-2-methyl-propyliden)-2-indolinon
   Hergestellt aus 1,5-Diacetyl-2-indolinon und 1,1,1-Trimethoxy-2-methylpropan
   Ausbeute: 40 % der Theorie
   R_{f} = 0,71 (Kieselgel, Essigester:Cyclohexan:Methanol 9:9:2)
   C₁₇H₁₉NO₄ (MG = 301,337)
   Massenspektrum: m/z = 302 (M+H)⁺
(7) 1,Acetyl-5-propionyl-3-(1-methoxy-propyliden)-2-indolinon
   Hergestellt aus 1-Acetyl-5-propionyl-2-indolinon und Orthopropionsäuretrimethylester

### Beispiel IV

| 1-Acetyl-5-butyryl-3-(ethoxy-phenyl-methyliden)-2-indolinon |
|---|
| 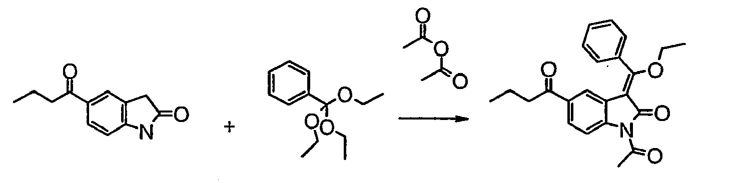 |

10 g (49 mmol) 5-Butyryl-2-indolinon (Bsp. I.2) werden in 200 ml Essigsäureanhydrid 5 h bei 130 °C gerührt. Dann werden 35 ml Orthobenzoesäuretriethylester zugegeben und weitere 4 h bei 100 °C gerührt. Anschließend wird eingeengt und der entstehende Niederschlag abgesaugt, gewaschen und getrocknet.
Ausbeute: 11,5 g (62 % der Theorie)
R_{f} = 0,79 (Kieselgel, Essigester/Cyclohexan/Methanol 9:9:2)
C₂₃H₂₃NO₄ (MG = 377,438)
Massenspektrum: m/z = 378 (M+H)⁺

Analog Beispiel IV werden folgende Verbindungen hergestellt:
(1) 1-Acetyl-5-isobutyryl-3-(ethoxy-phenyl-methyliden)-2-indolinon
   Hergestellt aus 5-Isobutyryl-2-indolinon, Essigsäureanhydrid und Orthobenzoesäuretriethylester
   R_{f} = 0,55 (Kieselgel, Essigester/Cyclohexan/Methanol 9:9:2)
(2) 1,5-Diacetyl-3-[1-methoxy-ethyliden]-2-indolinon 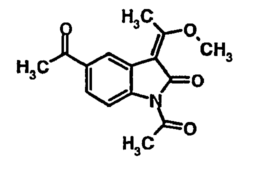
   Hergestellt aus 5-Acetyl-2-indolinon, Essigsäureanhydrid und Orthoessigsäuretrimethylester
   R_{f} = 0,40 (Kieselgel, Methylenchlorid/Methanol 50 :1)
(3) 1-Acetyl-5-propionyl-3-(ethoxy-phenyl-methyliden)-2-indolinon 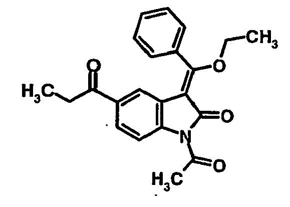
   Hergestellt aus 5-Propionyl-2-indolinon, Essigsäureanhydrid und Orthobenzoesäuretriethylester
   R_{f} = 0,79 (Kieselgel, Essigester/Cyclohexan/Methanol 9:9:2)
(4) 1-Acetyl-5-hexanoyl-3-(ethoxy-phenyl-methyliden)-2-indolinon 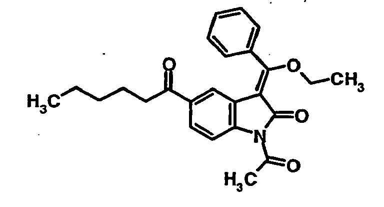
   Hergestellt aus 5-Hexanoyl-2-indolinon, Essigsäureanhydrid und Orthobenzoesäuretriethylester
   R_{f} = 0,72 (Methylenchlorid/Methanol 30 :1)
(5) 1-Acetyl-5-butyryl-3-(ethoxy-phenyl-methyliden)-2-indolinon 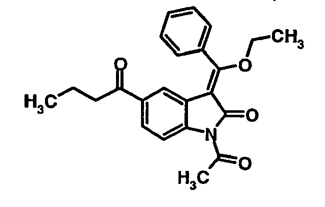
   Hergestellt aus 5-butyryl-2-indolinon, Essigsäureanhydrid und Orthobenzoesäuretriethylester
   R_{f} = 0,79 (Kieselgel, Essigester/Cyclohexan/Methanol 9:9:2)

### Beispiel V

| 1,5-Diacetyl-3-[(3,4-dimethoxy-phenyl)-hydroxy-methyliden]-2-indolinon |
|---|
| 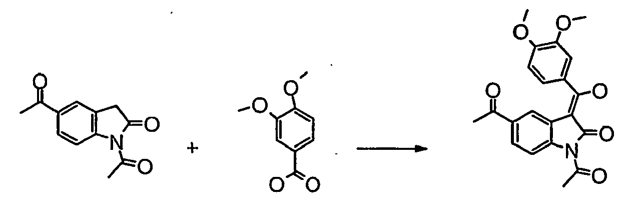 |

4,3 g (20 mmol) 1,5-Diacetyl-2-indolinon (Bsp. II) werden zusammen mit 4 g 3,4-Dimethoxybenzoesäure, 7,1 g TBTU (O-Benzotriazol-1-yl-N,N,N',N'-tetramethyluroniumtetrafluoroborat) und 14 ml Triethylamin in 80 ml DMF (Dimethylformamid) bei Raumtemperatur über Nacht gerührt. Dann wird der Ansatz auf 300 ml Eiswasser mit 10 ml konz. Salzsäure gegossen und der entstandene Niederschlag abgesaugt. Der Rückstand wird mit wenig Methanol und anschließend mit Ether gewaschen.
Ausbeute: 6,2 g (81,3 % der Theorie)
R_{f} = 0,85 (Kieselgel, Methylenchlorid/Methanol 9:1)
C₂₁H₁₉NO₆ (MG = 381,382)
Massenspektrum: m/z = 381 (M)⁺

Analog Beispiel V werden folgende Verbindungen hergestellt:
(1) 1,5-Diacetyl-3-[(benzo[1,3]dioxol-5-yl)-hydroxy-methyliden]-2-indolinon 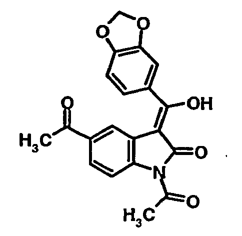
   Hergestellt aus 1,5-Diacetyl-2-indolinon und Piperonylsäure (Benzo[1,3]dioxol-5-carbonsäure)
   Ausbeute: 60 % der Theorie
   R_{f} = 0,70 (Kieselgel, Methylenchlorid/Methanol 9:1)
   C₂₀H₁₅NO₆ (MG = 365,339)
   Massenspektrum: m/z = 366 (M+H)⁺
(2) 1,5-Diacetyl-3-[(4-nitro-phenyl)-hydroxy-methyliden]-2-indolinon 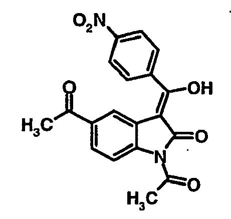
   Hergestellt aus 1,5-Diacetyl-2-indolinon und 4-Nitrobenzoesäure
   Ausbeute: 82 % der Theorie
   R_{f} = 0,38 (Kieselgel, Methylenchlorid/Methanol 9:1)
   C₁₉H₁₄N₂O₆ (MG = 366,328)
   Massenspektrum: m/z = 367 (M+H)⁺
(3) 1,5-Diacetyl-3-[(3-nitro-phenyl)-hydroxy-methyliden]-2-indolinon 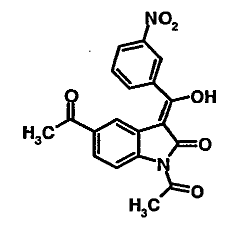
   Hergestellt aus 1,5-Diacetyl-2-indolinon und 3-Nitrobenzoesäure
   Ausbeute: 75 % der Theorie
   R_{f} = 0,38 (Kieselgel, Methylenchlorid/Methanol 9:1)
   C₁₉H₁₄N₂O₆ (MG = 366,328)
   Massenspektrum: m/z = 367 (M+H)⁺
(4) 1,5-Diacetyl-3-[(4-methoxycarbonyl-phenyl)-hydroxy-methyliden]-2-indolinon 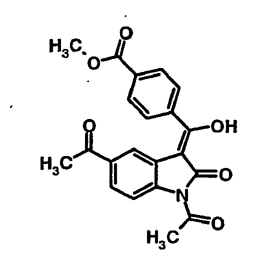
   Hergestellt aus 1,5-Diacetyl-2-indolinon und Terephthalsäuremonomethylester
   Ausbeute: 71 % der Theorie
   R_{f} = 0,41 (Kieselgel, Methylenchlorid/Methanol 30:1)
   C₂₁H₁₇NO₆ (MG = 379,366)
   Massenspektrum: m/z = 380 (M+H)⁺
(5) 1,5-Diacetyl-3-[(4-chloro-phenyl)-hydroxy-methyliden]-2-indolinon 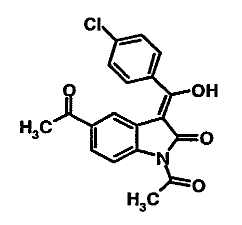
   Hergestellt aus 1,5-Diacetyl-2-indolinon und 4-Chlorobenzoesäure
   Ausbeute: 87 % der Theorie
   C₁₉H₁₄ClNO₄ (MG = 355,776)
   Massenspektrum: m/z = 356/358 (M+H)⁺
(6) 1,5-Diacetyl-3-[(3,4-dichlor-phenyl)-hydroxy-methyliden]-2-indolinon 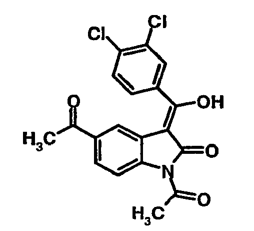
   Hergestellt aus 1,5-Diacetyl-2-indolinon und 3,4-Dichlorobenzoesäure
   Ausbeute: 83 % der Theorie
   C₁₉H₁₃Cl₂NO₄ (MG = 390,221)

   Massenspektrum: m/z = 390/392/394 (M+H)⁺
(7) 1,5-Diacetyl-3-[(4-cyano-phenyl)-hydroxy-methyliden]-2-indolinon 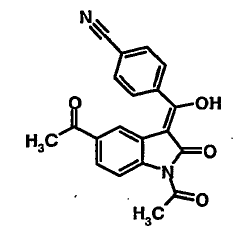
   Hergestellt aus 1,5-Diacetyl-2-indolinon und 4-Cyanobenzoesäure
   Ausbeute: 71 % der Theorie
   R_{f} = 0,32 (Kieselgel, Methylenchlorid/Methanol 9:1)
   C₂₀H₁₄N₂O₄ (MG = 346,341)
   Massenspektrum: m/z = 347 (M+H)⁺
(8) 1,5-Diacetyl-3-[(4-trifluoromethyl-phenyl)-hydroxy-methyliden]-2-indolinon 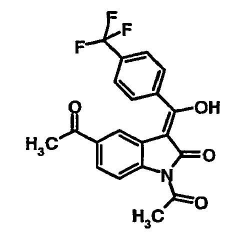
   Hergestellt aus 1,5-Diacetyl-2-indolinon und 4-Trifluoromethyl-benzoesäure
   Ausbeute: 83 % der Theorie
   C₂₀H₁₄F₃NO₄ (MG = 389,328)
   Massenspektrum: m/z = 390 (M+H)⁺
(9) 1,5-Diacetyl-3-[(2,3-dihydro-benzo-[1,4]dioxin-6-yl)-hydroxy-methyliden]-2-indolinon 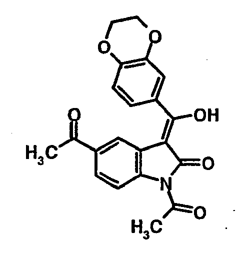
   Hergestellt aus 1,5-Diacetyl-2-indolinon und 2,3-Dihydro-1,4-benzodioxin-6-carbonsäure
   Ausbeute: 90 % der Theorie
   R_{f} = 0,75 (Kieselgel, Methylenchlorid/Methanol 9:1)
   C₂₁H₁₇NO₆ (MG = 379,366)
   Massenspektrum: m/z = 380 (M+H)⁺
(10) 1,5-Diacetyl-3-[(3-methoxy-phenyl)-hydroxy-methyliden]-2-indolinon 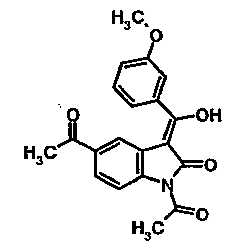
   Hergestellt aus 1,5-Diacetyl-2-indolinon und 3-Methocybenzoesäure
   Ausbeute: 70 % der Theorie
   R_{f} = 0,67 (Kieselgel, Methylenchlorid/Methanol 9:1)
(11) 1,5-Diacetyl-3-[(4-methoxy-phenyl)-hydroxy-methyliden]-2-indolinon 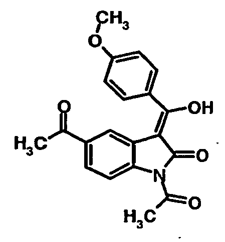
   Hergestellt aus 1,5-Diacetyl-2-indolinon und 4-Methocybenzoesäure
   Ausbeute: 59 % der Theorie
   R_{f} = 0,39 (Kieselgel, Methylenchlorid/Methanol 9:1)
   C₂₀H₁₇NO₅ (MG = 351,356)
   Massenspektrum: m/z = 350 (M-H)⁻
(12) 1-Diacetyl-5-propionyl-3-[(benzo[1,3]dioxol-5-yl)-hydroxy-methyliden]-2-indolinon 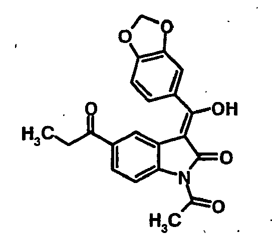
   Hergestellt aus 1-Acetyl-5-propionyl-2-indolinon und Piperonylsäure (Benzo[1,3]-dioxol-5-carbonsäure)
   Ausbeute: 67 % der Theorie
   R_{f} = 0,49 (Kieselgel, Methylenchlorid/Methanol 30:1)
   C₂₁H₁₇NO₆ (MG = 379,366)
   Massenspektrum: m/z = 380 (M+H)⁺
(13) 1,5-Diacetyl-3-[(4-bromophenyl)-hydroxy-methyliden]-2-indolinon 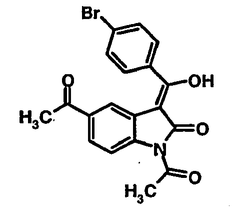
   Hergestellt aus 1,5-Diacetyl-2-indolinon und 4-Bromobenzoesäure
   Ausbeute: 89 % der Theorie
   C₁₉H₁₄BrNO₄ (MG = 400,227)
   Massenspektrum: m/z = 400/402 (M+H)⁺
(14) 1,5-Diacetyl-3-[(3,5-dichlor-phenyl)-hydroxy-methyliden]-2-indolinon 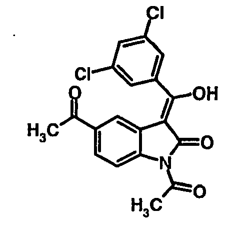
   Hergestellt aus 1,5-Diacetyl-2-indolinon und 3,5-Dichlorobenzoesäure
   Ausbeute: 79 % der Theorie
   R_{f} = 0,26 (Kieselgel, Methylenchlorid/Methanol 30:1)
   C₁₉H₁₃Cl₂NO₄ (MG = 390,221)
   Massenspektrum: m/z = 390/392/394 (M+H)⁺
(15) 1,5-Diacetyl-3-[(3,5-dimethoxyphenyl)-hydroxy-methyliden]-2-indolinon 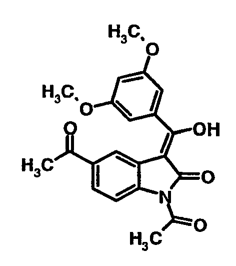
   Hergestellt aus 1,5-Diacetyl-2-indolinon und 3,5-Dimethoxybenzoesäure
   Ausbeute: 83 % der Theorie
   R_{f} = 0,37 (Kieselgel, Methylenchlorid/Methanol 30:1)
   C₂₁H₁₉NO₆ (MG = 381,382)
   Massenspektrum: m/z = 382 (M+H)⁺
(16) 1,5-Diacetyl-3-[(2-chloro-phenyl)-hydroxy-methyliden]-2-indolinon 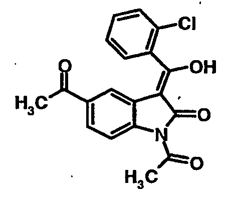
   Hergestellt aus 1,5-Diacetyl-2-indolinon und 2-Chlorobenzoesäure
   Ausbeute: 96 % der Theorie
   C₁₉H₁₄ClNO₄ (MG = 355,776)
   Massenspektrum: m/z = 356/358 (M+H)⁺
(17) 1,5-Diacetyl-3-[(2-methoxy-phenyl)-hydroxy-methyliden]-2-indolinon 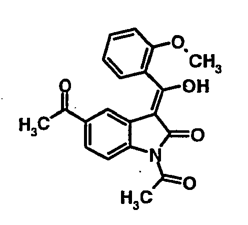
   Hergestellt aus 1,5-Diacetyl-2-indolinon und 2-Methocybenzoesäure
   Ausbeute: 27 % der Theorie
   C₂₀H₁₇NO₅ (MG = 351,356)
   Massenspektrum: m/z = 352 (M+H)⁺
(18) 1,5-Diacetyl-3-[(2,6-difluoro-phenyl)-hydroxy-methyliden]-2-indolinon 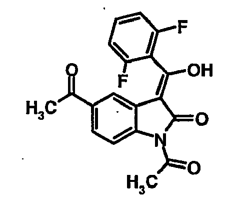
   Hergestellt aus 1,5-Diacetyl-2-indolinon und 2,6-Difluorbenzoesäure
   Ausbeute: 52 % der Theorie
   C₁₉H₁₃F₂NO₄ (MG = 357,311)
   Massenspektrum: m/z = 358 (M+H)⁺
(19) 1,5-Diacetyl-3'-[(4-fluorphenyl)-hydroxy-methyliden]-2-indolinon 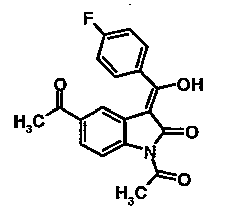
   Hergestellt aus 1,5-Diacetyl-2-indolinon und 4-Fluorbenzoesäure
   Ausbeute: 77 % der Theorie
   C₁₉H₁₄FNO₄ (MG = 339,321)
   Massenspektrum: m/z = 338 (M-H)⁻
(20) 1,5-Diacetyl-3-[(3,4-difluor-phenyl)-hydroxy-methyliden]-2-indolinon 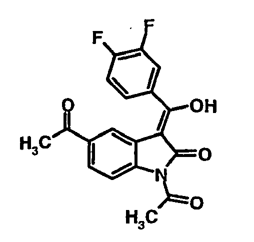
   Hergestellt aus 1,5-Diacetyl-2-indolinon und 3,4-Difluorbenzoesäure
   Ausbeute: 91 % der Theorie
(21) 1,5-Diacetyl-3-[(2,2-difluor-benzo[1,3]dioxol-5-yl)-hydroxy-methyliden]-2-indolinon 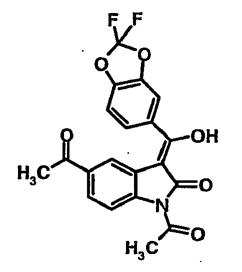
   Hergestellt aus 1,5-Diacetyl-2-indolinon und 2,2-Difluor-benzo[1,3]dioxol-5-carbonsäure
   Ausbeute: 69 % der Theorie
   C₂₀H₁₃F₂NO₆ (MG = 401,32)
   Massenspektrum: m/z = 402 (M+H)⁺
(22) 1,5-Diacetyl-3-[(4-(2-methoxycarbonyl-ethyl)-phenyl)-hydroxy-methyliden]-2-indolinon 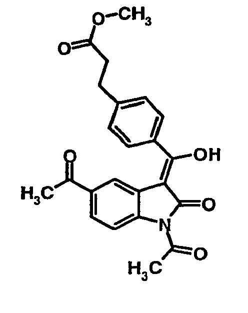
   Hergestellt aus 1,5-Diacetyl-2-indolinon und 4-(2-methoxycarbonyl-ethyl)-benzoesäure
   Ausbeute: 23 % der Theorie
   C₂₃H₂₁NO₆ (MG = 407,42)
   Massenspektrum: m/z = 408 (M+H)⁺
(23) 1,5-Diacetyl-3-[(pyrazin-2-yl)-hydroxy-methyliden]-2-indolinon 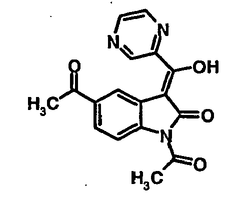
   Hergestellt aus 1,5-Diacetyl-2-indolinon und Pyrazin-2-carbonsäure
   Ausbeute: 57 % der Theorie
   C₁₇H₁₃N₃O₄ (MG = 323,311)
   Massenspektrum: m/z = 324 (M+H)⁺
(24) 1,5-Diacetyl-3-[(pyridin-4-yl)-hydroxy-methyliden]-2-indolinon 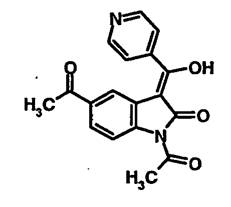
   Hergestellt aus 1,5-Diacetyl-2-indolinon und Isonikotinsäure (Pyridin-4-carbonsäure)
   Ausbeute: 87 % der Theorie
   C₁₈H₁₄N₂O₄ (MG = 322,323)
   Massenspektrum: m/z = 323 (M+H)⁺
(25) 1,5-Diacetyl-3-[(furan-3-yl)-hydroxy-methyliden]-2-indolinon 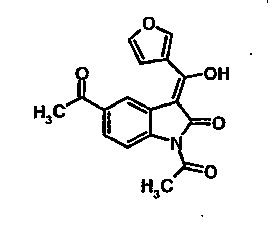
   Hergestellt aus 1,5-Diacetyl-2-indolinon und Furan-3-carbonsäure
   Ausbeute: 73 % der Theorie
   C₁₇H₁₃NO₅ (MG = 311,297)
   Massenspektrum: m/z = 312 (M+H)⁺
(26) 1,5-Diacetyl-3-[(4-diethylaminomethyl-phenyl)-hydroxy-methyliden]-2-indolinon 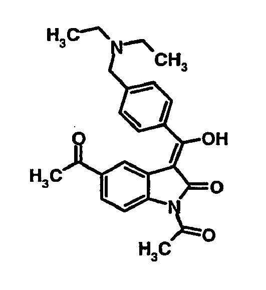
   Hergestellt aus 1,5-Diacetyl-2-indolinon und 4-Diethylaminomethyl-benzoesäure
   Ausbeute: 10 % der Theorie
   C₂₄H₂₆N₂O₄ (MG = 406,486)
   Massenspektrum: m/z = 407 (M+H)⁺
(27) 1,5-Diacetyl-3-[(4-methoxycarbonylmethoxy-phenyl)-hydroxy-methyliden]-2-indolinon 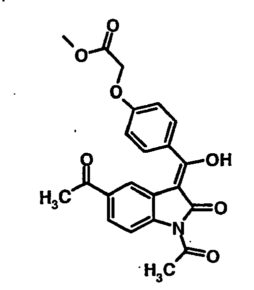
   Hergestellt aus 1,5-Diacetyl-2-indolinon und 4-Methoxycarbonyl-methoxybenzoesäure
   Ausbeute: 43 % der Theorie
   C₂₂H₁₉NO₇ (MG = 409,39)
   Massenspektrum: m/z = 410 (M+H)⁺
(28) 1,5-Diacetyl-3-[(4-methylsulfonyl-phenyl)-hydroxy-methyliden]-2-indolinon 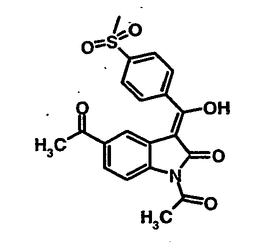
   Hergestellt aus 1,5-Diacetyl-2-indolinon und 4-Methylsulfonyl-benzoesäure
   Ausbeute: 25 % der Theorie
   C₂₀H₁₇NO₆S (MG = 399,418)
   Massenspektrum: m/z = 400 (M+H)⁺
(29) 1,5-Diacetyl-3-[(4-(2-diethylamino-ethoxy)-phenyl)-hydroxy-methyliden]-2-indolinon 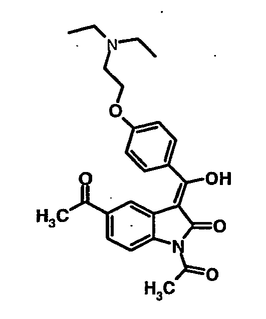
   Hergestellt aus 1,5-Diacetyl-2-indolinon und 4-Diethylamino-ethoxy-benzoesäure
   Ausbeute: 27 % der Theorie
   C₂₅H₂₈N₂O₅ (MG = 436,500)
   Massenspektrum: m/z = 437 (M+H)⁺
(30) 1,5-Diacetyl-3-[(3-(2-diethylamino-ethoxy)-phenyl)-hydroxy-methyliden]-2-indolinon 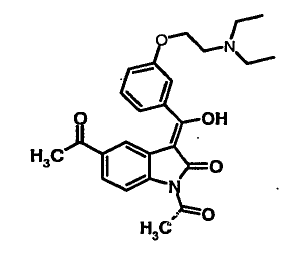
   Hergestellt aus 1,5-Diacetyl-2-indolinon und 3-Diethylamino-ethoxy-benzoesäure
   Ausbeute: 43 % der Theorie
   C₂₅H₂₈N₂O₅ (MG = 436,500)
   Massenspektrum: m/z = 437 (M+H)⁺
(31) 1,5-Diacetyl-3-[(3-(2-diethylamino-ethoxy)-phenyl)-hydroxy-methyliden]-2-indolinon 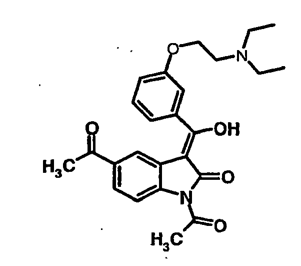
   Hergestellt aus 1,5-Diacetyl-2-indolinon und 3-Diethylamino-ethoxy-benzoesäure
   Ausbeute: 43 % der Theorie
   C₂₅H₂₈N₂O₅ (MG = 436,500)
   Massenspektrum: m/z = 437 (M+H)⁺
(31) 1,5-Diacetyl-3-[(3-(2-diethylamino-ethoxy)-phenyl)-hydroxy-methyliden]-2-indolinon 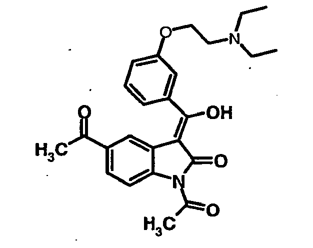
   Hergestellt aus 1,5-Diacetyl-2-indolinon und 3-Diethylamino-ethoxy-benzoesäure
   Ausbeute: 43 % der Theorie
   C₂₅H₂₈N₂O₅ (MG = 436,500)
   Massenspektrum: m/z = 437 (M+H)⁺
(32) 1,5-Diacetyl-3- (1-hydroxy-heptyliden)-2-indolinon 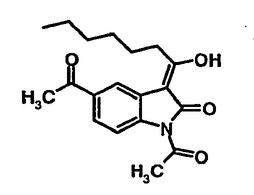
   Hergestellt aus 1,5-Diacetyl-2-indolinon und Heptansäure
(33) 1,5-Diacetyl-3- (1-hydroxy-hexyliden)-2-indolinon 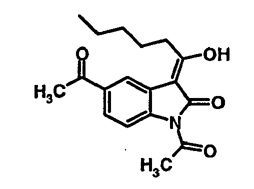
   Hergestellt aus 1,5-Diacetyl-2-indolinon und Hexansäure
(34) 1,5-Diacetyl-3- (1-hydroxy-3-methyl-butyliden)-2-indolinon 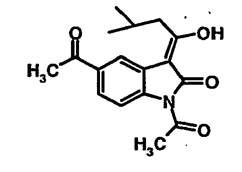
   Hergestellt aus 1,5-Diacetyl-2-indolinon und Isovaleriansäure

### Beispiel VI

| 1,5-Diacetyl-3-[(3,4-dimethoxy-phenyl)-methoxy-methyliden]-2-indolinon |
|---|
| 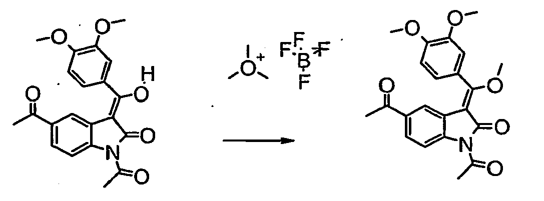 |

4,0 g (10,5 mmol) 1,5-Diacetyl-3-[(3,4-dimethoxy-phenyl)-hydroxy-methyliden]-2-indolinon (Bsp. V) werden in 100 ml Methylenchlorid suspendiert und mit 3,1 g (21 mmol) Trimethyloxoniumtetrafluorborat sowie 7,2 ml Hünigbase (Ethyldiisopropylamin) bei Raumtemperatur versetzt. Die Lösung wird 3 h gerührt, dann werden nochmals 1,55 g Trimethyloxoniumtetrafluorborat und 3,5 ml Hünigbase zugegeben und über Nacht gerührt. Nachdem nochmals dieselbe Reagenzmenge zugegeben und weitere 5 h gerührt wurde, wird die Umsetzung dreimal mit Wasser gewaschen, die organische Phase über Natriumsulfat getrocknet, filtriert und einrotiert. Der Rückstand wird über eine Kieselgelsäule mit Methylenchlorid/Methanol 9:1 chromatographiert, die entsprechende Fraktionen vereinigt und einrotiert.
Ausbeute: 1,6 g (37 % der Theorie)
R_{f} = 0,78 (Kieselgel, Methylenchlorid/Methanol 50:1)
C₂₂H₂₁NO₆ (MG = 395,409)
Massenspektrum: m/z = 396 (M+H)⁺

Analog Beispiel VI werden folgende Verbindungen hergestellt:
(1) 1,5-Diacetyl-3-[(benzo[1,3]dioxol-5-yl)-methoxy-methyliden]-2-indolinon 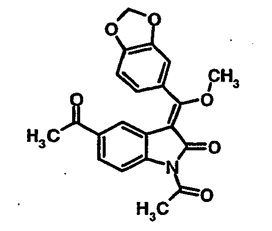
   Hergestellt aus 1,5-Diacetyl-3-[(benzo[1,3]dioxol-5-yl)-hydroxy-methyliden]-2-indolinon (Bsp. V.1)
   Ausbeute: 85 % der Theorie
   R_{f} = 0,55 (Kieselgel, Methylenchlorid/Methanol 30:1)
   C₂₁H₁₇NO₆ (MG = 379,366)
   Massenspektrum: m/z = 380 (M+H)⁺
(2) 1,5-Diacetyl-3-[(4-nitro-phenyl)-methoxy-methyliden]-2-indolinon 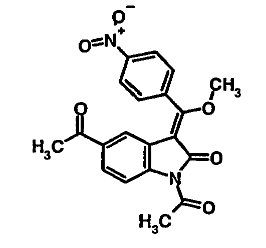
   Hergestellt aus 1,5-Diacetyl-3-[(4-nitro-phenyl)-hydroxy-methyliden]-2-indolinon (Bsp. V.2)
   Ausbeute: 82 % der Theorie
   R_{f} = 0,55 (Kieselgel, Methylenchlorid/Methanol 30:1)
   C₂₀H₁₆N₂O₆ (MG = 380,354 )
   Massenspektrum: m/z = 381 (M+H)⁺
(3) 1,5-Diacetyl-3-[(3-nitro-phenyl)-methoxy-methyliden]-2-indolinon 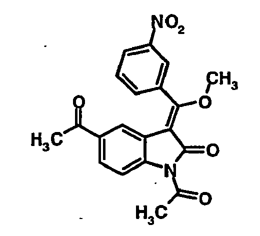
   Hergestellt aus 1,5-Diacetyl-3-[(3-nitro-phenyl)-hydroxy-methyliden]-2-indolinon (Bsp. V.3)
   Ausbeute: 43 % der Theorie
   R_{f} = 0,44 (Kieselgel, Methylenchlorid/Methanol 9:1)
   C₂₀H₁₆N₂O₆ (MG = 380,354)
   Massenspektrum: m/z = 381 (M+H)⁺
(4) 1,5-Diacetyl-3-[(4-methyloxycarbonyl-phenyl)-methoxy-methyliden]-2-indolinon 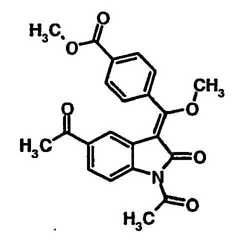
   Hergestellt aus 1,5-Diacetyl-3-[(4-methyloxycarbonyl-phenyl)-hydroxy-methyliden]-2-indolinon (Bsp. V.4)
   Ausbeute: 52 % der Theorie
   R_{f} = 0,56 (Kieselgel, Methylenchlorid/Methanol 30:1)
   C₂₂H₁₉NO₆ (MG = 393,393)
   Massenspektrum: m/z = 394 (M+H)⁺
(5) 1,5-Diacetyl-3-[(4-chloro-phenyl)-methoxy-methyliden]-2-indolinon 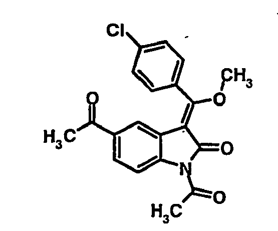
   Hergestellt aus 1,5-Diacetyl-3-[(4-chloro-phenyl)-hydroxy-methyliden]-2-indolinon (Bsp. V.5)
   Ausbeute: 65 % der Theorie
   C₂₀H₁₆ClNO₄ (MG = 369,802 )
   Massenspektrum: m/z = 370/372 (M+H)⁺
(6) 1,5-Diacetyl-3-[(3,4-dichlor-phenyl)-methoxy-methyliden]-2-indolinon 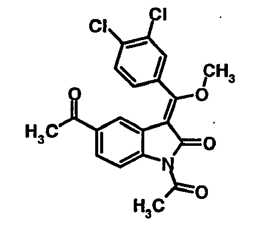
   Hergestellt aus 1,5-Diacetyl-3-[(3,4-dichlor-phenyl)-hydroxy-methyliden]-2-indolinon (Bsp. V.6)
   Ausbeute: 72 % der Theorie
   C₂₀H₁₅Cl₂NO₄ (MG = 404,247)
   Massenspektrum: m/z = 404/406/408 (M+H)⁺
(7) 1,5-Diacetyl-3-[(4-cyano-phenyl)-methoxy-methyliden]-2-indolinon 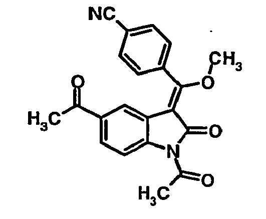
   Hergestellt aus 1,5-Diacetyl-3-[(4-cyano-phenyl)-hydroxy-methyliden]-2-indolinon (Bsp. V.7)
   Ausbeute: 53 % der Theorie
   C₂₁H₁₆N₂O₄ (MG = 360,367 )
   Massenspektrum: m/z = 361 (M+H)⁺
(8) 1,5-Diacetyl-3-[(4-trifluoromethyl-phenyl)-methoxy-methyliden]-2-indolinon 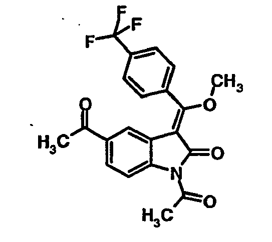
   Hergestellt aus 1,5-Diacetyl-3-[(4-trifluoromethyl-phenyl)-hydroxy-methyliden]-2-indolinon (Bsp. V.8)
   Ausbeute: 37 % der Theorie
   C₂₁H₁₆F₃NO₄ (MG = 403,354 )
   Massenspektrum: m/z = 404 (M+H)⁺
(9) 1,5-Diacetyl-3-[(2,3-dihydro-benzo-[1,4]dioxin-6-yl)-methoxy-methyliden]-2-indolinon 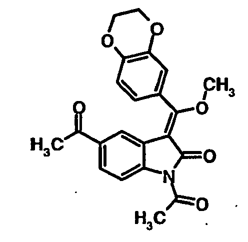
   Hergestellt aus 11,5-Diacetyl-3-[(2,3-dihydro-benzo-[1,4]dioxin-6-yl)-hydroxy-methyliden]-2-indolinon (Bsp. V.9)
   Ausbeute: 52 % der Theorie
   R_{f} = 0,82 (Kieselgel, Methylenchlorid/Methanol 9:1)
   C₂₂H₁₉NO₆ (MG = 393,393 )
   Massenspektrum: m/z = 394 (M+H)⁺
(10) 1,5-Diacetyl-3-[(3-methoxy-phenyl)-methoxy-methyliden]-2-indolinon 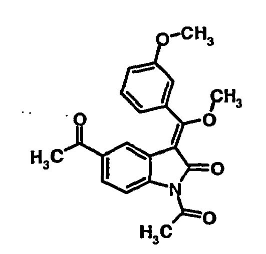
   Hergestellt aus 1,5-Diacetyl-3-[(3-methoxy-phenyl)-hydroxy-methyliden]-2-indolinon (Bsp. V.10)
   Ausbeute: 48 % der Theorie
   R_{f} = 0,40 (Kieselgel, Methylenchlorid/Methanol 9:1)
   C₂₁H₁₉NO₅ (MG = 365,383)
   Massenspektrum: m/z = 366 (M+H)⁺
(11) 1,5-Diacetyl-3-[(4-methoxy-phenyl)-methoxy-methyliden]-2-indolinon 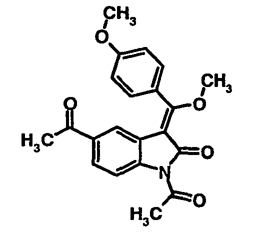
   Hergestellt aus 1,5-Diacetyl-3-[(4-methoxy-phenyl)-hydroxy-methyliden]-2-indolinon (Bsp.V.11)
   Ausbeute: 85% der Theorie
   R_{f} = 0,35 (Kieselgel, Methylenchlorid/Methanol 30:1)
   C₂₁H₁₉NO₅ (MG = 365,383 )
   Massenspektrum: m/z = 366 (M+H)⁺
(12) 1-Diacetyl-5-propionyl-3-[(benzo[1,3]dioxol-5-yl)-methoxy-methyliden]-2-indolinon 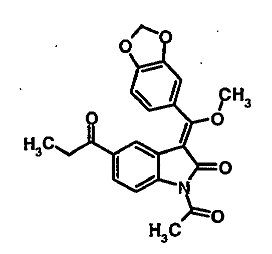
   Hergestellt aus 1-Diacetyl-5-propionyl-3-[(benzo[1,3]dioxol-5-yl)-hydroxy-methyliden]-2-indolinon (Bsp. V.12)
   Ausbeute: 98 % der Theorie
   R_{f} = 0,63 (Kieselgel, Methylenchlorid/Methanol 30:1)
   C₂₂H₁₉NO₆ (MG = 393,393 )
   Massenspektrum: m/z = 394 (M+H)⁺
(13) 1,5-Diacetyl-3-[(4-bromophenyl)-methoxy-methyliden]-2-indolinon 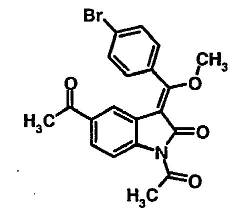
   Hergestellt aus 1,5-Diacetyl-3-[(4-bromophenyl)-hydroxy-methyliden]-2-indolinon (Bsp. V.13)
   Ausbeute: 48 % der Theorie
(14) 1,5-Diacetyl-3-[(3,5-dichlor-phenyl)-methoxy-methyliden]-2-indolinon 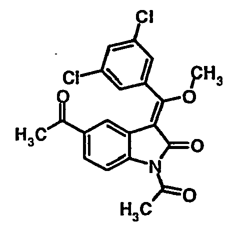
   Hergestellt aus 1,5-Diacetyl-3-[(3,5-dichlor-phenyl)-hydroxy-methyliden]-2-indolinon (Bsp.V.14)
   Ausbeute: 44 % der Theorie
   R_{f} = 0,86 (Kieselgel, Methylenchlorid/Methanol 30:1)
   C₁₉H₁₃Cl₂NO₄ (MG = 390,221 )
   Massenspektrum: m/z = 388/390/392 (Cl2, M+H)⁺
(15) 1,5-Diacetyl-3-[(3,5-dimethoxy-phenyl)-methoxy-methyliden]-2-indolinon 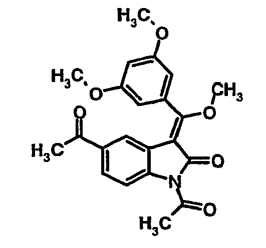
   Hergestellt aus 1,5-Diacetyl-3-[(3,5-dimethoxy-phenyl)-hydroxy-methyliden]-2-indolinon (Bsp. V.15)
   Ausbeute: 74 % der Theorie
   R_{f} = 0,65 (Kieselgel, Methylenchlorid/Methanol 30:1)
   C₂₂H₂₁NO₆ (MG = 395,409 )
   Massenspektrum: m/z = 396 (M+H)⁺
(16) 1,5-Diacetyl-3-[(2-chloro-phenyl)-methoxy-methyliden]-2-indolinon 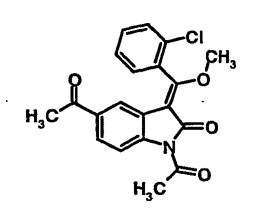
   Hergestellt aus 1,5-Diacetyl-3-[(2-chloro-phenyl)-hydroxy-methyliden]-2-indolinon (Bsp. V.16)
   Ausbeute: 54 % der Theorie
   C₂₀H₁₆ClNO₄ (MG = 369,802 )
   Massenspektrum: m/z = 370/372 (M+H)⁺
(17) 1,5-Diacetyl-3-[(2-methoxy-phenyl)-methoxy-methyliden]-2-indolinon 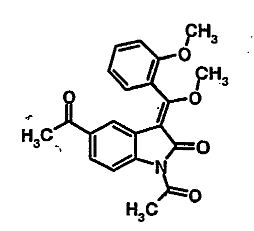
   Hergestellt aus 1,5-Diacetyl-3-[(2-methoxy-phenyl)-hydroxy-methyliden]-2-indolinon (Bsp. V.17)
   Ausbeute: 56 % der Theorie
   C₂₁H₁₉NO₅ (MG = 365,383)
   Massenspektrum: m/z = 366 (M+H)⁺
(18) 1,5-Diacetyl-3-[(2,6-difluoro-phenyl)-methoxy-methyliden]-2-indolinon 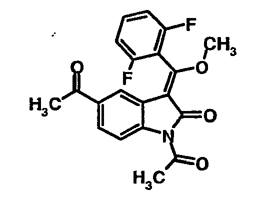
   Hergestellt aus 1,5-Diacetyl-3-[(2,6-difluoro-phenyl)-hydroxy-methyliden]-2-indolinon (Bsp.V.18)
   Ausbeute: 59 % der Theorie
   C₂₀H₁₅F₂NO₄ (MG = 3371,337 )
   Massenspektrum: m/z = 372 (M+H)⁺
(19) 1,5-Diacetyl-3-[(4-fluorphenyl)-methoxy-methyliden]-2-indolinon 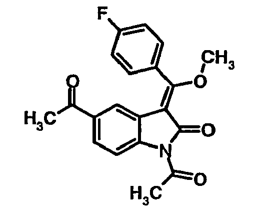
   Hergestellt aus 1,5-Diacetyl-3-[(4-fluorphenyl)-hydroxy-methyliden]-2-indolinon (Bsp. V.19)
   Ausbeute: 88 % der Theorie
   C₂₀H₁₆FNO₄ (MG = 353,347)
   Massenspektrum: m/z = 354 (M+H)⁺
(20) 1,5-Diacetyl-3-[(3,4-difluor-phenyl)-methoxy-methyliden]-2-indolinon 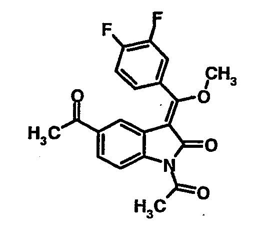
   Hergestellt aus 1,5-Diacetyl-3-[(3,4-difluor-phenyl)-hydroxy-methyliden]-2-indolinon (Bsp. V.20)
   Ausbeute: 23 % der Theorie
   C₂₀H₁₅F₂NO₄ (MG = 371,334)
   Massenspektrum: m/z = 372 (M+H)⁺
(21) 1,5-Diacetyl-3-[(2,2-difluor-benzo[1,3]dioxol-5-yl)-methoxy-methyliden]-2-indolinon 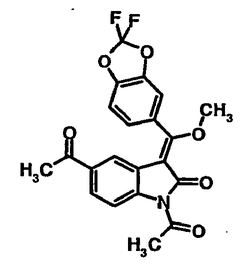
   Hergestellt aus 1,5-Diacetyl-3-[(2,2-difluor-benzo[1,3]dioxol-5-yl)-hydroxy-methyliden]-2-indolinon (Bsp. V.21)
   Ausbeute: 6 % der Theorie
   C₂₁H₁₅F₂NO₆ (MG = 415,346 )
   Massenspektrum: m/z = 416 (M+H)⁺
(22) 1,5-Diacetyl-3-[(4-(2-methoxycarbonyl-ethyl)-phenyl)-methoxy-methyliden]-2-indolinon 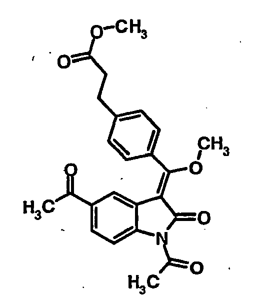
   Hergestellt aus 1,5-Diacetyl-3-[(4-(2-methoxycarbonyl-ethyl)-phenyl)-hydroxy-methyliden]-2-indolinon (Bsp. V.22)
   Ausbeute: 63 % der Theorie
   C₂₄H₂₃NO₆ (MG = 421,447)
   Massenspektrum: m/z = 422 (M+H)⁺
(23) 1,5-Diacetyl-3-[furan-3-yl-methoxy-methyliden]-2-indolinon 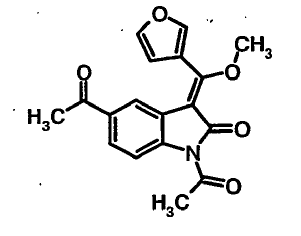
   Hergestellt aus 1,5-Diacetyl-3-[furan-3-yl-hydroxy-methyliden]-2-indolinon (Bsp. V.25) Ausbeute: 59 % der Theorie
   C₁₈H₁₅NO₅ (MG = 325,324 )
   Massenspektrum: m/z = 326 (M+H)⁺
(24) 1,5-Diacetyl-3-[(4-methoxycarbonylmethoxy-phenyl)-methoxy-methyliden]-2-indolinon 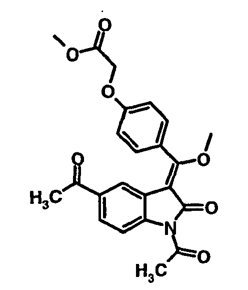
   Hergestellt aus 1,5-Diacetyl-3-[(4-methoxycarbonylmethoxy-phenyl)-hydroxy-methyliden]-2-indolinon
   Ausbeute: 24 % der Theorie
   C₂₃H₂₁NO₇ (MG = 423,415)
   Massenspektrum: m/z = 424 (M+H)⁺
(25) 1,5-Diacetyl-3-[(4-methylsulfonyl-phenyl)-methoxy-methyliden]-2-indolinon 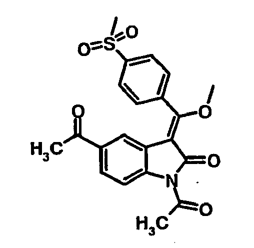
   Hergestellt aus 1,5-Diacetyl-3-[(4-methylsulfonyl-phenyl)-hydroxy-methyliden]-2-indolinon (Bsp. V.28)
   Ausbeute: 20 % der Theorie
   C₂₁H₁₉NO₆S (MG = 413,445)
   Massenspektrum: m/z = 414 (M+H)⁺
(26) 1,5-Diacetyl-3-(1-methoxy-octyliden)-2-indolinon 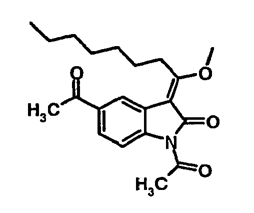
   Hergestellt aus 1,5-Diacetyl-3-(1-hydroxyl-octyliden)-2-indolinon (Bsp. VIII)
   Ausbeute: 82 % der Theorie
   C₂₁H₂₇NO₄S (MG = 357,443)
   Massenspektrum: m/z = 358 (M+H)⁺
(27) 1,5-Diacetyl-3-(1-methoxy-heptyliden)-2-indolinon 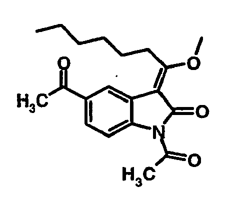
   Hergestellt aus 1,5-Diacetyl-3- (1-hydroxy-heptyliden)-2-indolinon (Bsp. V.32)
(28) 1,5-Diacetyl-3-(1-methoxy-hexyliden)-2-indolinon 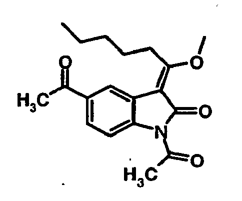
   Hergestellt aus 1,5-Diacetyl-3- (1-hydroxy-hexyliden)-2-indolinon (Bsp. V.33)
(29) 1,5-Diacetyl-3-(1-methoxy-3-methyl-butyliden)-2-indolinon 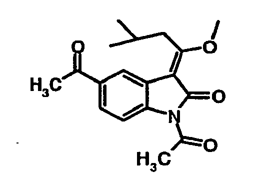
   Hergestellt aus 1,5-Diacetyl-3-(1-hydroxy-3-methyl-butyliden)-2-indolinon (Bsp. V.34)

### Beispiel VII

| 1,5-Diacetyl-3-[chloro-(pyrazin-2-yl)-methyliden]-2-indolinon |
|---|
| 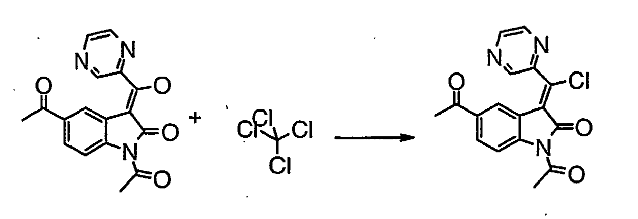 |

1,2 g (3,7 mmol) 1,5-Diacetyl-3-[pyrazin-2-yl-hydroxy-methyliden]-2-indolinon (Bsp. V.23) werden in 50 ml Dioxan gelöst und mit 2 ml Tetrachlorkohlenstoff und 2 g Triphenylphosphin 5 h am Rückfluß gekocht. Anschließend lässt man abkühlen und engt ein. Der Rückstand wird über eine Kieselgelsäule mit Methylenchlorid/Methanol 25:1 chromatographiert, die entsprechenden Fraktionen vereinigt und einrotiert.
Ausbeute: 400 mg (40 % der Theorie)
R_{f} = 0,70 (Kieselgel, Methylenchlorid/Methanol 30:1)
C₁₇H₁₂ClN₃O₃ (MG = 341,756)
Massenspektrum: m/z = 342/344 (M+H)⁺ (CL)

Analog Beispiel VII werden folgende Verbindungen hergestellt:
(1)1,5-Diacetyl-3-[chloro-(4-(2-dimethylamino-ethoxy)-phenyl)-methyliden]-2-indolinon

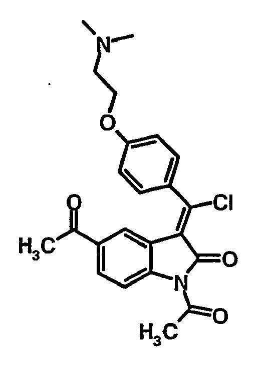

### Beispiel VIII

| 1,5-Diacetyl-3-(1-hydroxy-octyliden)-2-indolinon |
|---|
| 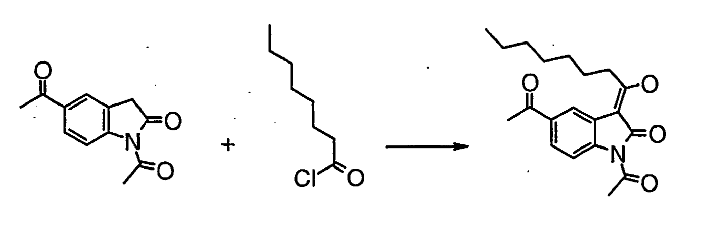 |

4,3 g (20 mmol) 1,5-Diacetyl-2-indolinon (Bsp. II) werden in 20 ml Dimethylformamid gelöst und 490 mg Dimethylaminopyridin (DMAP) und 6 ml Triethylamin zugegeben und im Eisbad gekühlt. Zu dieser Lösung gibt man 3,8 ml (22 mmol) Octansäurechlorid in 20 ml dimethylformamid und rührt weitere 10 min. Anschließend gibt man das Reaktionsgemisch in 150 ml Methylenchlorid und 150 ml 1 N Salzsäure. Die organische Phase wird abgetrennt, über Natriumsulfat getrocknet und einrotiert. Der Rückstand wird über eine Kieselgelsäule mit Methylenchlorid/Methanol 95:5 chromatographiert.
Ausbeute: 740 mg (11% der Theorie)
C₂₀H₂₅NO₄ (MG = 343,417)
Massenspektrum: m/z = 344 (M)⁺

### Herstellung der Endverbindungen:

Fließmittel:
   A: Methylenchlorid/Methanol 9:1
   B: Methalenchlorid/Methanol 4:1
   C: Methylenchlorid/Methanol/konz. Ammoniak 9:1:0,1
   D: Methylenchlorid/Methanoi 30:1
   E: Methylenchlorid/Methanol/Triethylamin 9:1:0,1

Bei den Formeln in der Tabelle stellt die freie gezeichnete Bindung stets die Bindung des jeweiligen Restes zum Anknüpfungspunkt im Molekül dar. Der Tabelleneintrag "-CH₃" bedeutet also einen Methylrest, der Eintrag 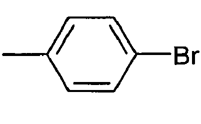 eine 4-Bromphenylgruppe

### Beispiel 1

| 5-Acetyl-3-[(4-dimethylaminomethylphenylamino)-phenyl-methyliden]-2-indolinon |
|---|
| 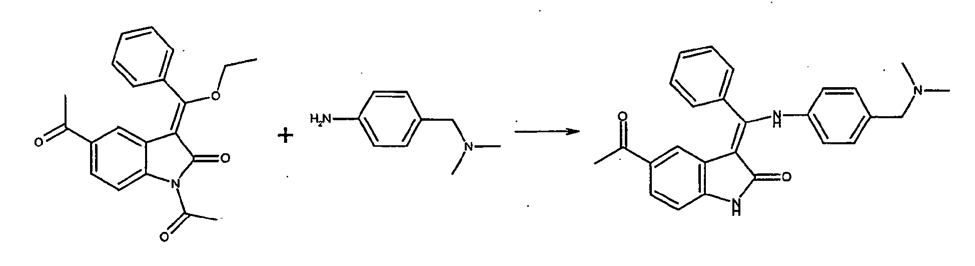 |

200 mg (0,57 mmol) 1,5-Diacetyl-3-[ethoxy-phenyl-methyliden]-2-indolinon (Bsp. III) werden in 5 ml Dimethylformamid suspendiert und mit 86 mg (0,57 mmol) 4-Dimethylaminomethyl-phenylamin bei 80°C Raumtemperatur etwa 5 h gerührt. Das acetylgeschützte Zwischenprodukt wird ohne Reinigung mit 2 ml konz. Ammoniak versetzt und man rührt bei Raumtemperatur über Nacht. Anschließend wird eingeengt und der Rückstand über eine Kieselgelsäule mit dem Fließmittel Methylenchlorid/Methanol 9:1 chromatographiert.
Ausbeute: 100 mg (42 % der Theorie)
R_{f} = 0,27 (Kieselgel, Methylenchlorid/Methanol 9:1)
C₂₆H₂₅N₃O₂ (MG = 411,502 )
Massenspektrum: m/z = 412 (M+H)⁺

Analog Beispiel 1 werden folgende Verbindungen der Formel I hergestellt:

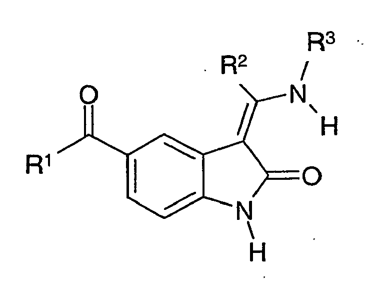

| Beispiel | R¹ | R² | R³ | Edukt Ausbeute [%] | Massenspektrum (ES) m/z | R_{f}-Wert (Kieselgel) (Fließmittel) |
|---|---|---|---|---|---|---|
| 1.001 | Me | Ph | 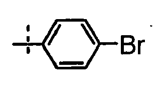 | III 60,5 | (M+H)⁺= 432/434 (Br) | 0,6 (EE /Cyclohexan/ MeOH 9:9:2) |
| 1.002 | Me | Ph | 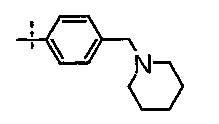 | III 35,0 | (M+H)⁺= 451 | 0,4 (A) |
| 1.003 | Me | Ph | 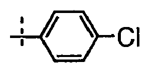 | III 56,7 | (M-H)⁻= 387 | nicht bestimmt |
| 1.004 | Me | Ph | 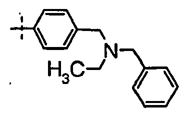 | III 74,9 | (M+H)⁺= 502 | 0,2 (D) |
| 1.005 | Me | Ph | 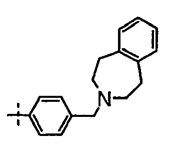 | III 56,2 | (M+H)⁺= 514 | 0,38 (A) |
| 1.006 | Me | Ph | 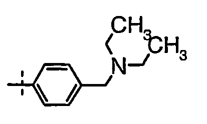 | III 47,7 | (M+H)⁺= 440 | 0,3 (A) |
| 1.007 | Me | Ph | 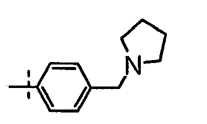 | III 47,9 | (M+H)⁺= 438 | 0.31 (A) |
| 1.008 | Me | Ph | 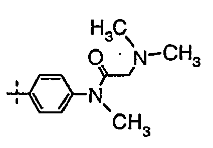 | III 20,5 | (M+H)⁺= 469 | 0,24 (A) |
| 1.009 | Et | Ph | 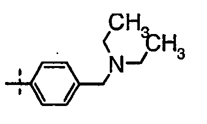 | IV.3 55,1 | (M+H)⁺= 454 | 0,3 (A) |
| 1.010 | Et | Ph | 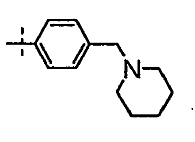 | IV.3 14,6 | (M-H)⁻= 464 | 0,33 (A) |
| 1.011 | Et | Ph | 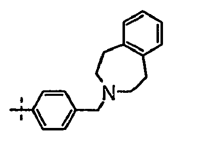 | IV.3 24,1 | (M+H)⁺= 528 | 0,33 (A) |
| 1.012 | Et | Ph | 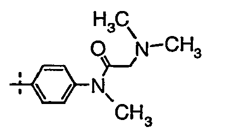 | IV.3 28,0 | (M+H)⁺= 483 | 0,27 (A) |
| 1.013 | n-C₅H₁₁ | Ph | 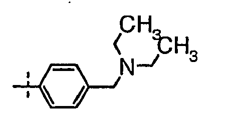 | IV.4 53,2 | (M-H)⁻= 494 | 0,32 (A) |
| 1.014 | n-C₅H₁₁ | Ph | 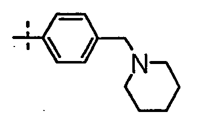 | IV.4 79,9 | (M-H)⁻= 506 | 0,28 (A) |
| 1.015 | n-C₅H₁₁ | Ph | 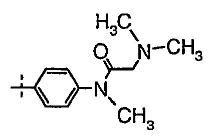 | IV.4 54,1 | (M-H)⁻= 523 | 0,33 (A) |
| 1.016 | Me | Ph | 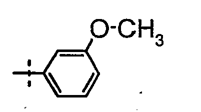 | III 30,8 | (M-H)⁻= 383 | 0,32 (A) |
| 1.017 | Me | Ph | 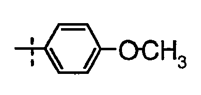 | III 57,0 | (M-H)⁻= 383 | 0,26 (A) |
| 1.018 | Me | Ph | 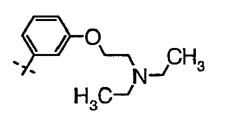 | III 53,0 | (M-H)⁻ = 468 | 0,19 (A) |
| 1.019 | Me | Ph | 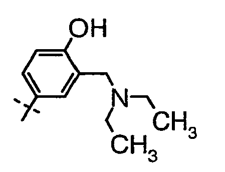 | III 9,8 | (M-H)⁻= 454 | 0,37 (A) |
| 1.020 | Me | Ph | 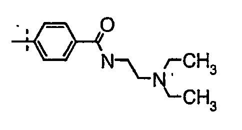 | III 35,5 | (M+H)⁺= 497 | 0,39 (B) |
| 1.021 | Me | Ph | 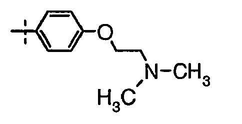 | III 70,0 | (M-H)⁻= 440 | 0,15 (A) |
| 1.022 | Ph | Ph | 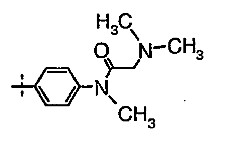 | III.2 28,4 | (M+H)⁺= 531 | 0,46 (A) |
| 1.023 | Ph | Ph | 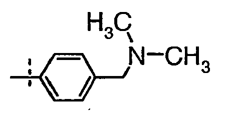 | III.2 46,3 | (M+H)⁺= 474 | 0,45 (A) |
| 1.024 | Ph | Ph | 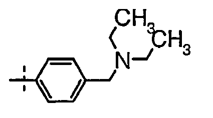 | III.2 46,5 | (M+H)⁺= 502 | 0,48 (A) |
| 1.025 | Ph | Ph | 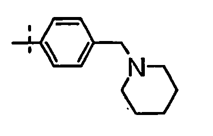 | III.2 57,4 | nicht bestimmt | 0,46 (A) |
| 1.026 | n-Pr | Ph | 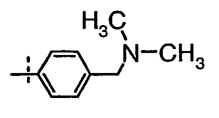 | IV.5 33,1 | (M+H)⁺= 440 | 0,32 (A) |
| 1.027 | n-Pr | Ph | 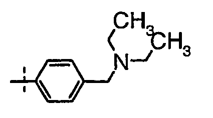 | IV.5 32,3 | (M+H)⁺= 468 | 0,34 (A) |
| 1.028 | n-Pr | Ph | 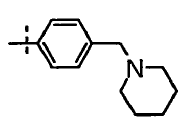 | IV.5 83,4 | (M+H)⁺= 480 | 0,36 (A) |
| 1.029 | n-Pr | Ph | 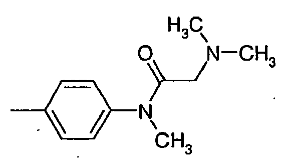 | IV.5 45,6 | (M+H)⁺= 497 | 0,36 (A) |
| 1.030 | i-Pr | Ph | 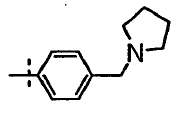 | IV.1 29,7 | (M-H)⁻= 464 | 0,3 (A) |
| 1.031 | i-Pr | Ph | 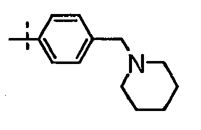 | IV.1 44,6 | (M+H)⁺= 480 | 0,25 (A) |
| 1.032 | i-Pr | Ph | 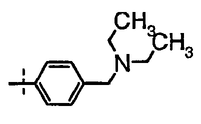 | IV.1 9,4 | (M+H)⁺= 468 | 0,28 (A) |
| 1.033 | i-Pr | Ph | 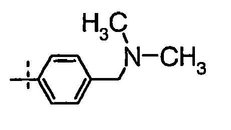 | IV.1 22,9 | (M+H)⁺= 440 | 0,31 (A) |
| 1.034 | i-Pr | Ph | 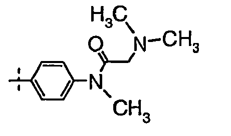 | VI.1 20,3 | (M+H)⁺= 497 | 0,28 (A) |
| 1.035 | Me | Ph | 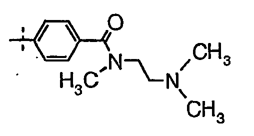 | III 53,1 | (M+H)⁺= 483 | 0,68 (A) |
| 1.036 | Me | Ph | 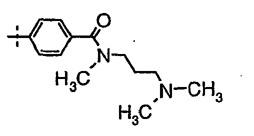 | III 42,2 | (M+H)⁺= 543 | 0,23 (B) |
| 1.037 | Me | Ph | 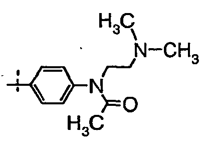 | III 33,8 | (M+H)⁺= 483 | 0,6 (B) |
| 1.038 | Me | Ph | 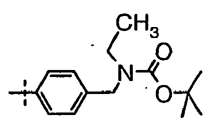 | III 59,2 | (M+H)⁺= 512 | 0,86 (B) |
| 1.039 | Me | 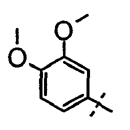 | 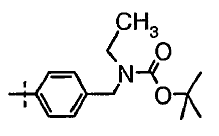 | VI 84,7 | (M+H)⁺= 572 | 0,87 (B) |
| 1.040 | Me | 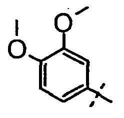 | 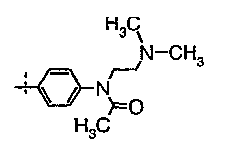 | VI 40,1 | (M+H)⁺= 543 | 0,43 (B) |
| 1.041 | Me | 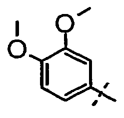 | 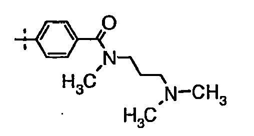 | VI 17,8 | (M+H)⁺= 557 | 0,36 (B) |
| 1.042 | Me | 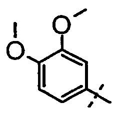 | 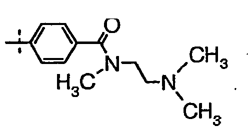 | VI 16,4 | (M+H)⁺= 543 | 0,35 (B) |
| 1.043 | Me | 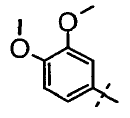 | 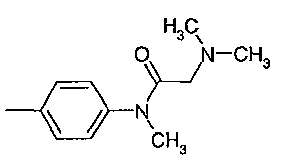 | VI 61,7 | (M+H)⁺= 529 | 0,38 (B) |
| 1.044 | Me | Ph | 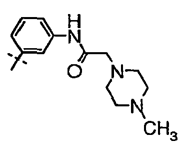 | III 32,1 | (M+H)⁺= 510 | 0,34 (C) |
| 1.045 | Me | Ph | 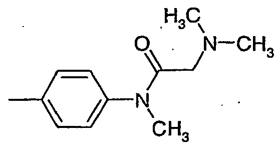 | III 66,9 | (M+H)⁺= 469 | 0,46 (C) |
| 1.046 | Me | Ph | 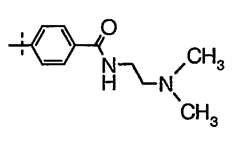 | III 60,8 | (M+H)⁺= 483 | 0,32 (C) |
| 1.047 | Me | Ph | 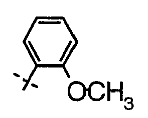 | III 81,9 | (M+H)⁺= 385 | 0,57 (A) |
| 1.048 | Me | 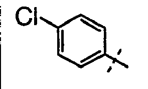 | 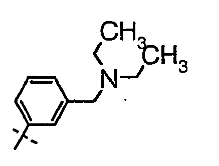 | VI.5 74,9 | (M+H)⁺= 474/476 (Cl) | 0,26 (A) |
| 1.049 | Me | 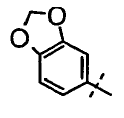 | 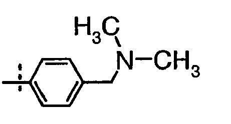 | VI.1 39,2 | (M+H)⁺= 456 | 0,33 (A) |
| 1.050 | Me | 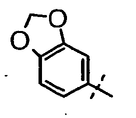 | 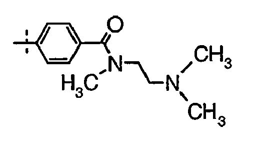 | VI.2 6,2 | (M+H)⁺= 527 | 0,61 (B) |
| 1.051 | Me | 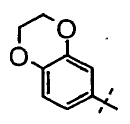 | 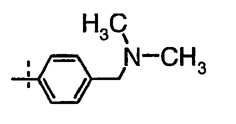 | VI.9 60,8 | (M+H)⁺= 470 | 0,4 (A) |
| 1.052 | Me | 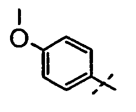 | 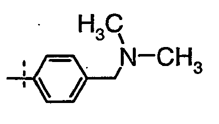 | VI.11 29,0 | (M-H)⁻= 440 | 0,05 (A) |
| 1.053 | Me | 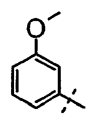 | 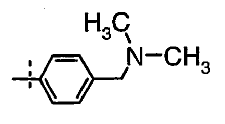 | VI.10 29,0 | (M+H)⁺= 442 | 0,24 (A) |
| 1.054 | Me | 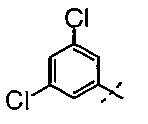 | 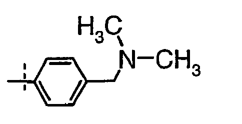 | VI.14 46,3 | (M+H)⁺= 480/482/ 484 (Cl2) | 0,33 (A) |
| 1.055 | Me | 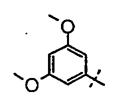 | 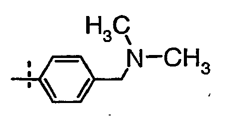 | VI.15 58,7 | (M+H)⁺= 472 | 0,15 (A) |
| 1.056 | Me | 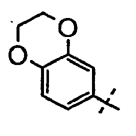 | 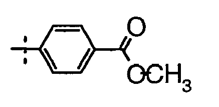 | VI.9 75,2 | (M+H)⁺= 471 | 0,42 (A) |
| 1.057 | Me | 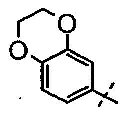 | 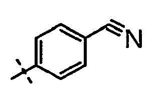 | VI.9 46,8 | (M+H)⁺= 438 | 0,44 (A) |
| 1.058 | Me | 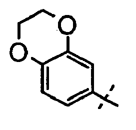 | 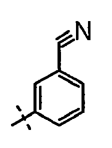 | VI.9 25,2 | (M+H)⁺= 438 | 0,38 (A) |
| 1.059 | Me | 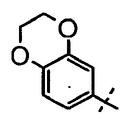 | 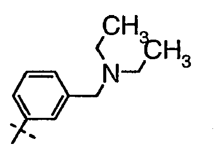 | VI.9 74,7 | (M+H)⁺= 498 | 0,27(A) |
| 1.060 | Me | 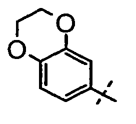 | 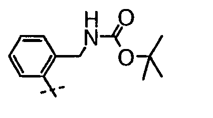 | VI.9 39,9 | (M+H)⁺= 542 | 0,47 (A) |
| 1.061 | Me | 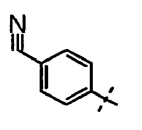 | 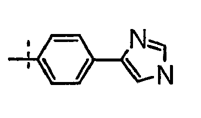 | VI.7 69,9 | (M+H)⁺= 446 | 0,34 (A) |
| 1.062 | Me | 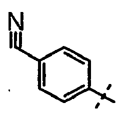 | 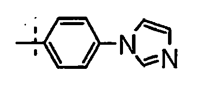 | VI.7 58,2 | (M+H)⁺= 446 | 0,23 (A) |
| 1.063 | Me | 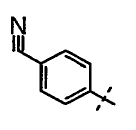 | 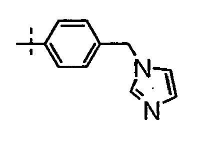 | VI.7 47,0 | (M+H)⁺= 460 | 0,38 (A) |
| 1.064 | Me | 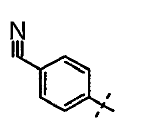 | 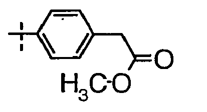 | VI.7 70,2 | (M-H)⁻= 450 | 0,52 (A) |
| 1.065 | Me | 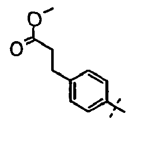 | 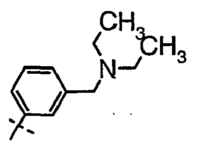 | VI.22 48,1 | (M+H)⁺= 526 | 0,37 (A) |
| 1.066 | Me | 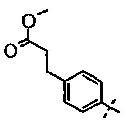 | 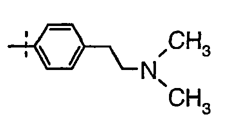 | VI.22 65,9 | (M+H)⁺= 512 | 0,22 (A) |
| 1.067 | Me | Ph | 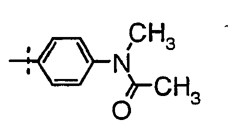 | III 82,3 | (M+H)⁺= 426 | 0,6 (A) |
| 1.068 | Me | 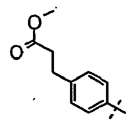 | 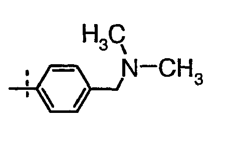 | VI.22 56,5 | (M+H)⁺= 498 | 0,36 (A) |
| 1.069 | Et | 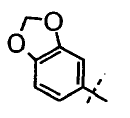 | 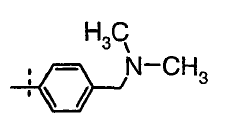 | VI.12 32,3 | (M+H)⁺= 470 | 0,13 (A) |
| 1.070 | Me | Me | 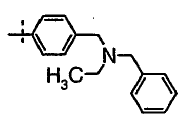 | IV.2 83,9 | (M+H)⁺= 440 | 0,80 (A) |
| 1.071 | Me | Me | 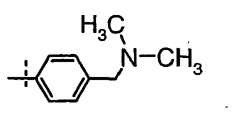 | IV.2 73,0 | (M+H)⁺= 350 | 0,33 (A) |
| 1.072 | Me | Et | 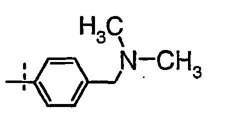 | III.3 49,0 | (M+H)⁺= 364 | 0,33 (E) |
| 1.073 | Me | n-Pr | 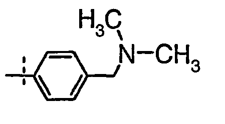 | III.4 21,0 | (M+H)⁺= 378 | 0,52 (C) |
| 1.074 | Me | Ph | 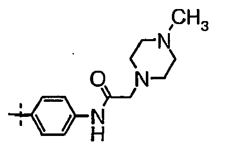 | III 56,0 | (M+H)⁺= 510 | 0,51 (C) |
| 1.075 | Me | Ph | 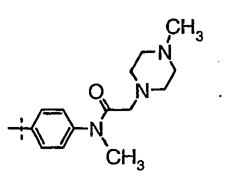 | III 21,0 | (M+H)⁺= 524 | 0,49 (C) |
| 1.076 | Me | 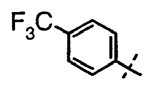 | 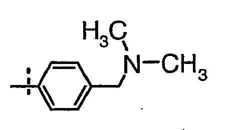 | VI.8 64,0 | (M+H)⁺= 480 | 0,32 (A) |
| 1.077 | Me | n-Bu | 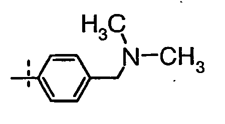 | III.5 38,0 | (M+H)⁺= 390 | 0,45 (E) |
| 1.078 | Me | 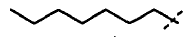 | 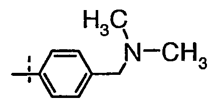 | VI.26 62,6 | (M+H)⁺= 432 | 0,30 (A) |

### Beispiel 2

| 5-Acetyl-3-[(4-ethylaminomethyl-phenylamino)-phenyl-methyliden]-2-indolinon-triflat |
|---|
| 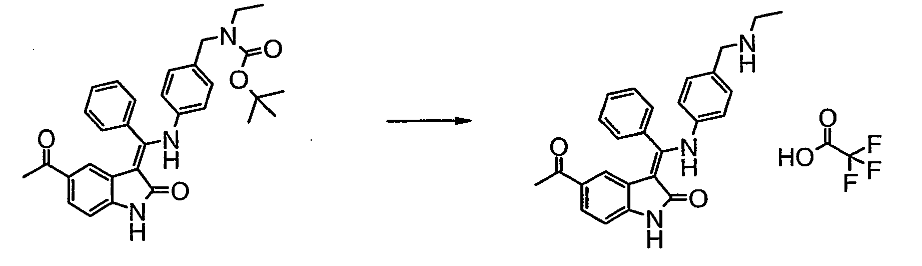 |

250 mg (0.49 mmol) 5-Acetyl-3-[(4-((tert-butoxycarbonyl-ethyl-amino)-methyl)-pnehyl-amino-phenyl-methyliden]-2-indolinon (Beispiel 1.038) werden in 10 ml Methylen-chlorid gelöst und portionsweise mit 5 ml Trifluoresigsäure versetzt. Anschließend läßt man die Lösung 3 h bei Raumtemperatur rühren. Dann wird zur Trockene einrotiert und der Rückstand mit Methanol gewaschen.
Ausbeute : 300 mg (97% der Theorie)
R_{f} = 0,37 (Kieselgel, Methylenchlorid/Methanol 4:1)
C₂₆H₂₅N₃O₂ (MG = 411,51)
Massenspektrum : m/z = 412 (M+H)⁺

Analog Beispiel 2 werden folgende Verbindungen der Formel I, jeweils als Triflate, hergestellt :

| Beispiel | R¹ | R² | R³ | Edukt-Ausbeute [%] | Massenspektrum (ES) m/z | Rf-Wert(Kieselgel) (Fließmittel) |
|---|---|---|---|---|---|---|
| 2.001 | Me | 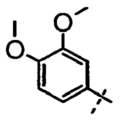 | 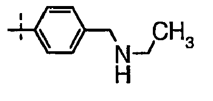 | 1.039 93,1 | (M+H)⁺ = 472 | 0,39 (B) |
| 2.002 | Me | 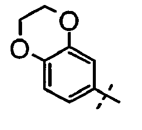 | 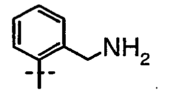 | 1.060 97,5 | (M+H)⁺ = 442 | 0,21 (A) |

### Beispiel 3

| 5-Acetyl-3-{[4-(2-carboxy-ethyl)-phenyl]-[4-(dimethylaminomethyl)-phenylamino]-methyliden}-2-indolinon |
|---|
| 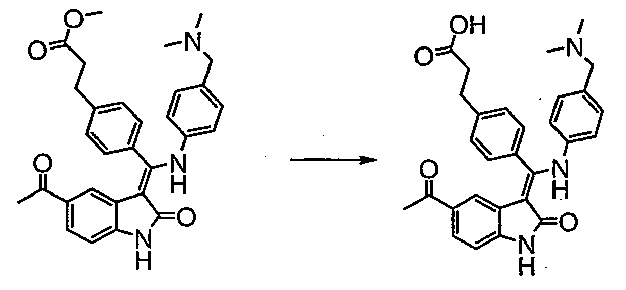 |

100 mg (0,20 mmol) 5-Acetyl-3-{[4-(2-methoxycarbonyl-ethyl)-phenyl]-[4-(dimethylaminomethyl)-phenylamino]-methyliden}-2-indolinon (Beispiel 1.068) werden in 0,4 ml 1 N Natronllauge und 4 ml Methanol suspendiert und über Nacht gerührt. Anschließend läßt man abkühlen. gibt 0,8 ml 1 N Salzsäure zu und engt zur Trockene ein. Den Rückstand nimmt man in 20 ml Methylenchlorid/Methanol 30:1 auf, trocknet über Natriumsulfat und engt ein.
Ausbeute: 96 mg (98 % der Theorie)
R_{f} = 0,50 (Kieselgel, Methanol)
C₂₉H₂₉N₃O₄ (MG = 483,565)
Massenspektrum: m/z = 484 (M+H)⁺

Analog Beispiel 3 werden folgende Verbindungen der Formel I hergestellt:

| Beispiel | R¹ | R² | R³ | Edukt Ausbeute [%] | Massenspektrum (ES) m/z | R_{f}-Wert (Kieselgel) (Rießmittel) |
|---|---|---|---|---|---|---|
| 3.001 | Me | 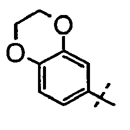 | 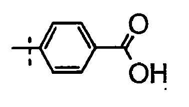 | 1.055 92,8 | (M+H)⁺= 457 | 0,27 (A) |
| 3.002 | Me | 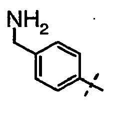 | 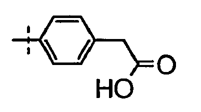 | 4.002 45,0 | (M-H)⁻= 440 | 0,47 (MeOH) |
| 3.003 | Me | 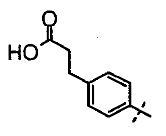 | 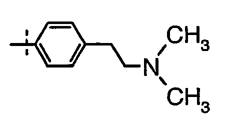 | 1.066 94,8 | (M+H)⁺= 498 | 0,29 (MeOH) |
| 3.004 | Me | 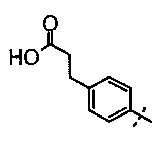 | 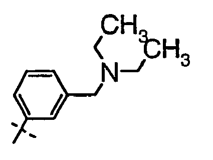 | 1.065 98 | (M+H)⁺= 512 | 0,31 (MeOH) |

### Beispiel 4

| 5-Acetyl-3-[(4-aminomethyl-phenyl)-(4-(methoxycarbonylmethyl)-phenylamino)-methyliden]-2-indolinon |
|---|
| 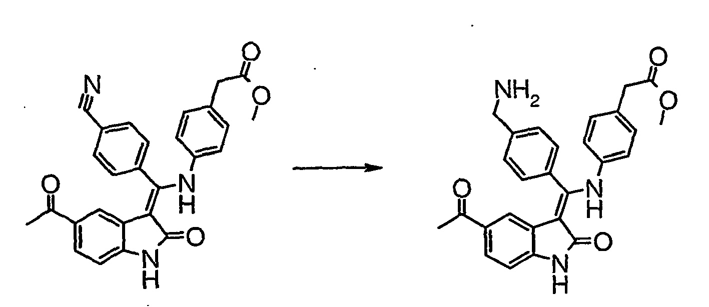 |

100 mg (0,22 mmol) 5-Acetyl-3-[(4-cyano-phenyl)-(4-(methoxycarbonylmethyl)-phenylamino)-methyliden]-2-indolinon (Beispiel 1.064) werden in 12 ml methanolischem Ammoniak gelöst, mit 80 mg Raneynickel versetzt und bei Raumtemperatur 7 h bei einem Druck von 50 psi hydriert. Anschließend wird der Katalysator abfiltriert und die Lösung eingeengt. Der Rückstand wird über eine Kieselgelsäule mit Methylenchlorid:Methanol 30:1 chromatographiert. Die gewünschte Fraktion wird gesammelt und eingeengt.
Ausbeute: 10 mg (9 % der Theorie)
R_{f} = 0,54 (Kieselgel, Methylenchlorid/Methanol/konz. Ammoniak 9:1:0,1)
C₂₇H₂₅N₃O₄ (MG = 455,512)
Massenspektrum: m/z = 456 (M+H)⁺

Analog Beispiel 4 werden folgende Verbindungen der Formel I hergestellt:

| Beispiel | R¹ | R² | R³ | Edukt Ausbeute [%] | Massenspektrum (ES) m/z | R_{f}-Wert (Kieselgel) (Fließmittel) |
|---|---|---|---|---|---|---|
| 4.001 | Me | 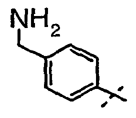 | 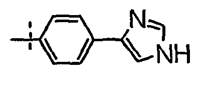 | 1.061 35,7 | (M+H)⁺= 450 | 0,28 (C) |
| 4.002 | Me | 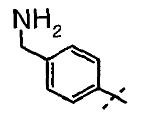 | 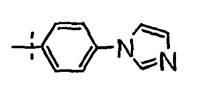 | 1.062 11,6 | (M+H)⁺= 450 | 0,31 (C) |

### Beispiel 5

| 5-Acetyl-3-[(4-acetylamino-methyl-phenyl)-(4-(methoxycarbonylmethyl)-phenyl-amino)-methyliden]-2-indolinon |
|---|
| 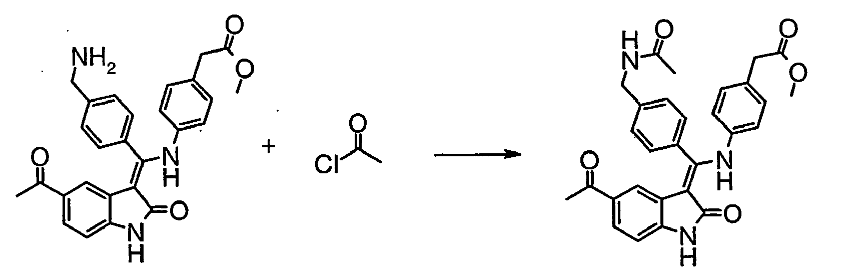 |

80 mg (0,17 mmol) 5-Acetyl-3-[(4-aminomethyl-phenyl)-(4-(methoxycarbonylmethyl)-phenylamino)-methyliden]-2-indolinon (Beispiel 4) werden in 4 ml Methylenchlorid vorgelegt und mit 75 µl Triethylamin versetzt. Zu dieser Lösung tropft man unter Eiskühlung 40 µl Acetylchlorid und läßt 10 min nachrühren. Anschließend lässt man den Ansatz auf Raumtemperatur erwärmen und rührt 5 h nach. Dann wir die Lösung mit Wasser gewaschen, die organsiche Phase über Natriumsulfat gewaschen, abgesaugt und einrotiert.
Ausbeute: 46 mg (52 % der Theorie)
R_{f} = 0,53 (Kieselgel, Methylenchlorid/Methanol 9:1)
C₂₉H₂₇N₃O₅ (MG = 497,548)
Massenspektrum: m/z = 496 (M-H)⁻

Analog Beispiel 5 wird folgende Verbindung der Formel I hergestellt:

| Beispiel | R¹ | R² | R³ | Edukt Ausbeute [%] | Massenspektrum (ES) m/z | R_{f}-Wert (Kieselgel) (Fließmittel) |
|---|---|---|---|---|---|---|
| 5.001 | Me | 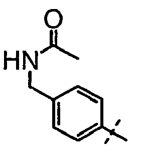 | 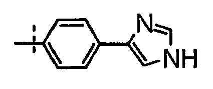 | 4.001 53,5 | (M+H)⁺= 492 | 0,20 (C) |
| 5.002 | Me | 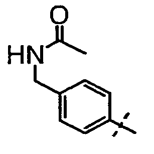 | 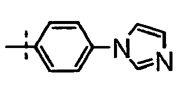 | 4.002 64,7 | (M+H)⁺= 492 | 0,41 (C) |

### Beispiel 6

### Dragées mit 75 mg Wirksubstanz

| 1 | Dragéekern enthält: | |
|---|---|---|
| | Wirksubstanz | 75,0 mg |
| | Calciumphosphat | 93,0 mg |
| | Maisstärke | 35,5 mg |
| | Polyvinylpyrrolidon | 10,0 mg |
| | Hydroxypropylmethylcellulose | 15,0 mg |
| | Magnesiumstearat | 1.5 mg |
| | | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt. Dragéegewicht: 245 mg.

### Beispiel 7

### Tabletten mit 100 mg Wirksubstanz

| Zusammensetzung: | | |
|---|---|---|
| 1 | Tablette enthält: | |
| | Wirksubstanz | 100,0 mg |
| | Milchzucker | 80,0 mg |
| | Maisstärke | 34,0 mg |
| | Polyvinylpyrrolidon | 4,0 mg |
| | Magnesiumstearat | 2,0 mg |
| | | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 8

### Tabletten mit 150 mg Wirksubstanz

| Zusammensetzung: | | |
|---|---|---|
| 1 | Tablette enthält: | |
| | Wirksubstanz | 150,0 mg |
| | Milchzucker pulv. | 89,0 mg |
| | Maisstärke | 40,0 mg |
| | Kolloide Kieselgelsäure | 10,0 mg |
| | Polyvinylpyrrolidon | 10,0 mg |
| | Magnesiumstearat | 1,0 mg |
| | | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 9

### Hartgelatine-Kapsein mit 150 mg Wirksubstanz

| 1 | Kapsel enthält: | |
|---|---|---|
| | Wirkstoff | 150,0 mg |
| | Maisstärke getr. | ca. 180,0 mg |
| | Milchzucker pulv. | ca. 87,0 mg |
| | Magnesiumstearat | 3,0 mg |
| | | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 10

### Suppositorien mit 150 mg Wirksubstanz

| 1 | Zäpfchen enthält: | |
|---|---|---|
| | Wirkstoff | 150,0 mg |
| | Polyethylenglykol 1500 | 550,0 mg |
| | Polyethylenglykol 6000 | 460,0 mg |
| | Polyoxyethylensorbitanmonostearat | 840,0 mg |
| | | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 11

### Suspension mit 50 mg Wirksubstanz

| 100 ml Suspension enthalten: | | |
|---|---|---|
| | Wirkstoff | 1,00 g |
| | Carboxymethylcellulose-Na-Salz | 0,10 g |
| | p-Hydroxybenzoesäuremethylester | 0,05 g |
| | p-Hydroxybenzoesäurepropylester | 0,01 g |
| | Rohrzucker | 10,00 g |
| | Glycerin | 5,00 g |
| | Sorbitlösung 70%ig | 20,00 g |
| | Aroma | 0,30 g |
| | Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 12

### Ampullen mit 10 mg Wirksubstanz

| Zusammensetzung: | | |
|---|---|---|
| | Wirkstoff | 10,0 mg |
| | 0,01 n Salzsäure s.q. | |
| | Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 13

### Ampullen mit 50 mg Wirksubstanz

| Zusammensetzung: | | |
|---|---|---|
| | Wirkstoff | 50,0 mg |
| | 0,01 n Salzsäure s.q. | |
| | Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 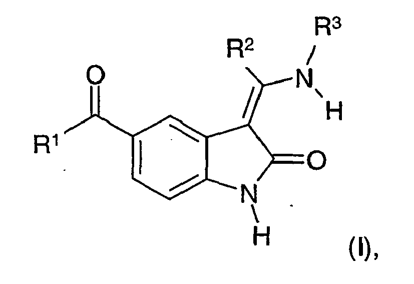 in der
R¹ eine lineare oder verzweigte C₁₋₅-Alkylgruppe, in der die Wasserstoffatome ganz oder teilweise durch Fluoratome ersetzt sein können, oder
eine gegebenenfalls durch ein Fluor-, Chlor- oder Bromatom substiuierte Arylgruppe,
wobei unter einer Arylgruppe eine Phenyl- oder Naphthylgruppe zu verstehen ist,
R² eine C₁₋₇-Alkyl- oder C₃₋₇-Cycloalkylgruppe,
eine gegebenenfalls durch ein oder zwei Fluor-, Chlor-, Brom- oder lodatome oder ein oder zwei Nitro-, Cyano-, Amino-, C₁₋₃-Alkyl- oder C₁₋₃-Alkoxygruppen substituierte 5- oder 6-gliedrige Heteroarylgruppe mit ein bis drei Heteroatomen ausgewählt aus der Gruppe N, S und O, wobei sowohl die Heteroatome als auch die Substituenten gleich oder verschieden sein können,
eine Phenylgruppe, in der zwei benachbarte Kohlenstoffatome über eine Methylendioxy-, Ethylendioxy- oder Difluormethylendioxygruppe miteinander verknüpft sind, eine Phenylgruppe, an die ein weiterer Phenylring oder ein 5- oder 6-gliedriger heteroaromatischer Ring mit ein bis drei Heteroatomen ausgewählt aus der Gruppe N, S und O, wobei die Heteroatome gleich oder verschieden sein können, angeliert ist, und wobei der Bicyclus durch ein oder zwei Fluor-, Chlor-, Brom- oder lodatome oder ein oder zwei Nitro-, Cyano-, Amino-, C₁₋₃-Alkyl- oder C₁₋₃-Alkoxygruppen substituiert sein kann und die Substituenten gleich oder verschieden sein können, oder
eine Phenylgruppe, die durch ein bis drei Fluor-, Chlor-, Brom- oder lodatome oder durch eine bis drei C₁₋₃-Alkyl-, Nitro-, Cyano-, Amino-, Di-(C₁₋₃-alkyl)-amino-, C₁₋₃-Alkyl-carbonylamino-, Phenylcarbonylamino-, C₁₋₃-Alkylsulfonylamino-, Arylsulfonylamino-, Trifluormethyl-, C₁₋₃Alkylsulfonyl-, Carboxy-, C₁₋₃-Alkoxy-, Di-(C₁₋₃-alkyl)-amino-C₁₋₃-alkyloxy-, C₁₋₃-Alkoxy-carbonyl-, C₁₋₃-Alkylaminocarbonyl-, Hydroxycarbonyl-C₁₋₃-alkyl-aminocarbonyl-, C₁₋₃-Alkoxycarbonyl-C₁₋₃-alkyl-aminocarbonyl-, Di-(C₁₋₃-alkyl)-amino-C₁₋₃-alkylaminocarbonyl-, Di-(C₁₋₃-alkyl)-amino-carbonyl-C₁₋₃-alkoxy-, C₁₋₃-Alkyl-amino-carbonyl-C₁₋₃-alkoxy-, Carboxy-C₁₋₃-alkoxy-, C₁₋₃-Alkyloxy-carbonyl-C₁₋₃-alkoxy-, Carboxy-C₁₋₃-alkyl-, C₁₋₃-Alkoxy-carbonyl-C₁₋₃-alkyl-, C₁₋₃-Alkoxy-carbonylamino-C₁₋₃-alkyl-, Amino-C₁₋₃-alkyl-, Di-(C₁₋₃-alkyl)-amino-C₁₋₃-alkyl-, C₁₋₃-Alkyl-carbonylamino-C₁₋₃-alkyl-, Phthalimido-, Pyrrolyl- oder Mono- oder Di-(C₁₋₃-alkyl)-pyrrolylgruppen substituiert sein kann, wobei die Substituenten gleich oder verschieden sind, und
R³ eine Phenyl-, Napthyl- oder wie oben definierte Hetroarylgruppe, die
durch ein Fluor-, Chlor-, Brom- oder Jodatom,
durch eine Cyano-, Hydroxy-, Carboxy-, C₁₋₃-Alkoxy-, C₁₋₃-Alkoxycarbonyl-oder Di-(C₁₋₃-alkyl)-amino-C₁₋₃-alkoxygruppe,
durch eine C₁₋₃-Alkylgruppe, die durch eine Hydroxycarbonyl-, C₁₋₃-Alkoxycarbonyl- oder Heteroaryl)gruppe substituiert sein kann,
durch eine C₁₋₃-Alkylgruppe, die durch eine 3- bis 7-gliedrige Cyclcoalkyleniminogruppe substituiert ist, wobei an die Cycloalkyleniminogruppe über zwei benachbarte Kohlenstoffatome ein Benzolring ankondensiert sein kann,
durch eine Amino-C₁₋₃-alkylgruppe, die am Stickstoffatom durch eine oder zwei C₁₋₃-Alkyl-, Phenyl-C₁₋₃-alkyl- oder C₁₋₄-Alkoxy-carbonylgruppen substituiert sein kann, wobei die Substituenten gleich oder verschieden sind,
durch eine C₁₋₃-Alkyl-carbonyl-aminogruppe, die am Stickstoffatom durch eine C₁₋₃-Alkylgruppe oder eine endständig durch eine Di-(C₁₋₃-alkyl)-aminogruppe substituierte C₂₋₃-Alkylgruppe und im Alkylteil durch eine Di-(C₁₋₃-alkyl)-amino-, Piperazinyl- oder 4-(C₁₋₃-Alkyl)-piperazin-1-ylgruppe substituiert sein kann,
durch eine im Alkylteil endständig durch eine Di-(C₁₋₃-alkyl)-aminogruppe substituierte C₂₋₃-Alkyl-aminocarbonylgruppe, die zusätzlich am Stickstoffatom durch eine C₁₋₃-Alkylgruppe substituiert sein kann,
oder durch eine Heteroarylgruppe
mono-, di- oder trisubstituiert sein kann, wobei die Substituenten gleich oder verschieden sein können, bedeuten,
wobei die vorstehend erwähnten Alkylgruppen geradkettig oder verzweigt sein können,
deren Tautomere, Enantiomere, Diastereomere, deren Gemische und deren Salze.

2. Verbindungen der allgemeinen Formel I gemäß Anspruch 1, in denen
R² und R³ wie in Anspruch 1 erwähnt definiert sind und
R¹ eine Methyl-, Ethyl-, n-Propyl-, Isopropyl-, n-Pentyl-, Trifluormethyl- oder Phenylgruppe bedeutet,
deren Tautomere, Enantiomere, Diastereomere, deren Gemische und deren Salze.

3. Verbindungen der allgemeinen Formel I gemäß Anspruch 2, in denen
R¹ eine Methyl-, Ethyl-, n-Propyl-, Isopropyl-, n-Pentyl- oder Phenylgruppe,
R² eine C₁₋₇-Alkylgruppe,
eine Phenylgruppe, in der zwei benachbarte Kohlenstoffatome über eine Methylendioxy-, Ethylendioxy- oder Difluormethylendioxygruppe miteinander verknüpft sind, oder
eine Phenylgruppe, die durch ein oder zwei Fluor-, Chlor-, Brom- oder lodatome oder durch eine oder zwei C₁₋₃-Alkyl-, Nitro-, Cyano-, Amino-, C₁₋₃-Alkylcarbonylamino-, Phenylcarbonylamino-, C₁₋₃-Alkylsulfonylamino-, Trifluormethyl-, Carboxy-, C₁₋₃-Alkoxy-, Di-(C₁₋₃-alkyl)-amino-C₁₋₃-alkyloxy-, C₁₋₃-Alkoxy-carbonyl-, C₁₋₃-Alkylamino-carbonyl-, Hydroxycarbonyl-C₁₋₃-alkyl-aminocarbonyl-, C₁₋₃-Alkoxycarbonyl-C₁₋₃-alkyl-aminocarbonyl-, Di-(C₁₋₃-alkyl)-amino-C₁₋₃-alkylaminocarbonyl-, Carboxy-C₁₋₃-alkyl-, C₁₋₃-Alkoxy-carbonyl-C₁₋₃-alkyl-, Amino-C₁₋₃-alkyl- oder C₁₋₃-Alkyl-carbonylamino-C₁₋₃-alkylgruppen substituiert sein kann, wobei die Substituenten gleich oder verschieden sind, und
R³ eine Phenylgruppe, die
durch ein Fluor-, Chlor- oder Bromatom,
durch eine Cyano-, Hydroxy-, Carboxy-, C₁₋₃-Alkoxy-, C₁₋₃-Alkoxycarbonyl-oder Di-(C₁₋₃-alkyl)-amino-C₁₋₃-alkoxygruppe,
durch eine C₁₋₃-Alkylgruppe, die durch eine Hydroxycarbonyl-, C₁₋₃-Alkoxycarbonyl- oder Imidazolylgruppe substituiert sein kann,
durch eine C₁₋₃-Alkylgruppe, die durch eine 3- bis 7-gliedrige Cyclcoalkyleniminogruppe substituiert ist, wobei an die Cycloalkyleniminogruppe über zwei benachbarte Kohlenstoffatome ein Benzolring ankondensiert sein kann,
durch eine Amino-C₁₋₃-alkylgruppe, die am Stickstoffatom durch eine oder zwei C₁₋₃-Alkyl-, Benzyl- oder C₁₋₄-Alkoxy-carbonylgruppen substituiert sein kann, wobei die Substituenten gleich oder verschieden sind,
durch eine C₁₋₃-Alkyl-carbonyl-aminogruppe, die am Stickstoffatom durch eine C₁₋₃-Alkylgruppe oder eine endständig durch eine Di-(C₁₋₃-alkyl)-aminogruppe substituierte C₂₋₃-Alkylgruppe und im Alkylteil durch eine Di-(C₁₋₃-alkyl)-amino-, Piperazinyl- oder 4-(C₁₋₃-Alkyl)-piperazin-1-ylgruppe substituiert sein kann,
durch eine im Alkylteil endständig durch eine Di-(C₁₋₃-alkyl)-aminogruppe substituierte C₂₋₃-Alkyl-aminocarbonylgruppe, die zusätzlich am Stickstoffatom durch eine C₁₋₃-Alkylgruppe substituiert sein kann,
oder durch eine Imidazolylgruppe
mono- oder disubstituiert sein kann, wobei die Substituenten gleich oder verschieden sein können, bedeuten,
deren Tautomere, Enantiomere, Diastereomere, deren Gemische und deren Salze.

4. Verbindungen der allgemeinen Formel I gemäß Anspruch 3, in denen
R¹ eine Methylgruppe,
R² eine Ethyl-, Propyl-, Butyl- oder Pentylgruppe,
eine Phenylgruppe, in der zwei benachbarte Kohlenstoffatome über eine Methylendioxy-, Ethylendioxy- oder Difluormethylendioxygruppe miteinander verknüpft sind, oder
eine Phenylgruppe, die durch ein oder zwei Fluor-, Chlor-, Bromatome oder durch eine oder zwei C₁₋₃-Alkyl-, Cyano-, C₁₋₃-Alkoxy-, Carboxy-C₁₋₃-alkyl-, C₁₋₃-Alkoxycarbonyl-C₁₋₃-alkyl-, Amino-C₁₋₃-alkyl- oder C₁₋₃-Alkyl-carbonylamino-C₁₋₃-alkylgruppen substituiert sein kann, wobei die Substituenten gleich oder verschieden sind, und
R³ eine Phenylgruppe, die
durch ein Fluor-, Chlor- oder Bromatom,
durch eine Cyano-, Carboxy-, C₁₋₃-Alkoxy- oder C₁₋₃-Alkoxycarbonylgruppe,
durch eine C₁₋₃-Alkylgruppe, die durch eine Hydroxycarbonyl- oder C₁₋₃-Alkoxy-carbonylgruppe substituiert sein kann,
durch eine C₁₋₃-Alkylgruppe, die durch eine 3- bis 7-gliedrige Cyclcoalkyleniminogruppe substituiert ist,
durch eine Amino-C₁₋₃-alkylgruppe, die am Stickstoffatom durch eine oder zwei C₁₋₃-Alkyl- oder C₁₋₄-Alkoxy-carbonylgruppen substituiert sein kann, wobei die Substituenten gleich oder verschieden sind,
durch eine C₁₋₃-Alkyl-carbonyl-aminogruppe, die am Stickstoffatom durch eine C₁₋₃-Alkylgruppe oder eine endständig durch eine Di-(C₁₋₃-alkyl)-aminogruppe substituierte C₂₋₃-Alkylgruppe und im Alkylteil durch eine Di-(C₁₋₃-alkyl)-amino-oder 4-(Methyl)-piperazin-1-ylgruppe substituiert sein kann,
oder durch eine im Alkylteil endständig durch eine Di-(C₁₋₃-alkyl)-aminogruppe substituierte C₂₋₃-Alkyl-aminocarbonylgruppe, die zusätzlich am Stickstoffatom durch eine C₁₋₃-Alkylgruppe substituiert sein kann,
monosubstituiert oder durch eine Hydroxy- und eine Di-(C₁₋₃-alkyl)-amino-C₁₋₃-alkylgruppe disubstituiert sein kann, wobei die Substituenten gleich oder verschieden sein können, bedeuten,
wobei die vorstehend erwähnten Alkylgruppen geradkettig oder verzweigt sein können,
deren Tautomere, Enantiomere, Diastereomere, deren Gemische und deren Salze.

5. Verbindungen der allgemeinen Formel I gemäß Anspruch 4, in denen
R¹ eine Methylgruppe,
R² eine Phenylgruppe, in der zwei benachbarte Kohlenstoffatome über eine Methylendioxy- oder Ethylendioxygruppe miteinander verknüpft sind, oder
eine Phenylgruppe, die durch ein oder zwei Methoxygruppen substituiert sein kann, und
R³ eine eine Phenylgruppe, die
durch eine Cyanogruppe oder
durch eine Amino-C₁₋₃-alkylgruppe, die am Stickstoffatom durch eine oder zwei C₁₋₃-Alkylgruppen substituiert sein kann, wobei die Substituenten gleich oder verschieden sein können,
substituiert ist, bedeuten,
wobei die vorstehend erwähnten Alkylgruppen geradkettig oder verzweigt sein können,
deren Tautomere, Enantiomere, Diastereomere, deren Gemische und deren Salze;

6. Folgende Verbindungen der allgemeinen Formel I gemäß Anspruch 1:
(a) 5-Acetyl-3-{[4-(diethylaminomethyl)-phenylamino]-phenyl-methyliden}-2-indolinon 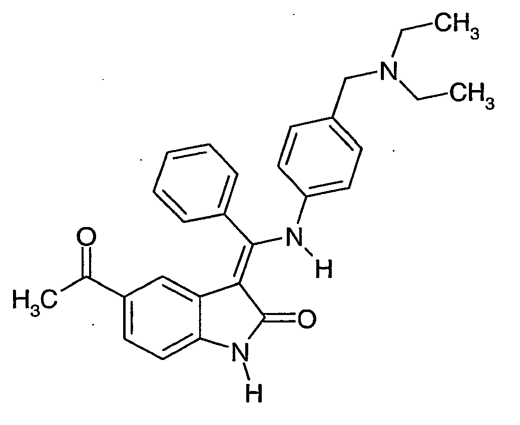
(b) 5-Acetyl-3-{[4-(dimethylamino-methyl)-phenylamino]-(2,3-dihydro-benzo[1,4]-dioxin-6-yl)-methyliden}-2-indolinon 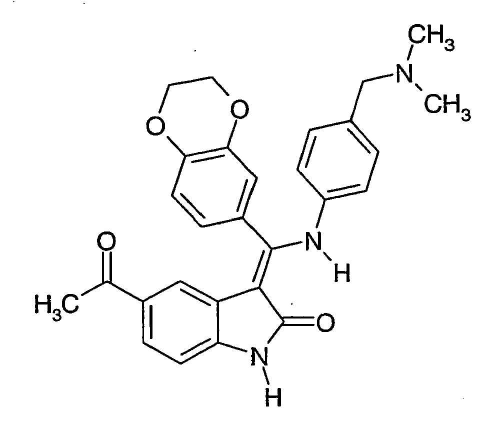
(c) 5-Acetyl-3-{[4-(dimethylaminomethyl)-phenylamino]-(3-methoxy-phenyl)-methyliden}-2-indolinon 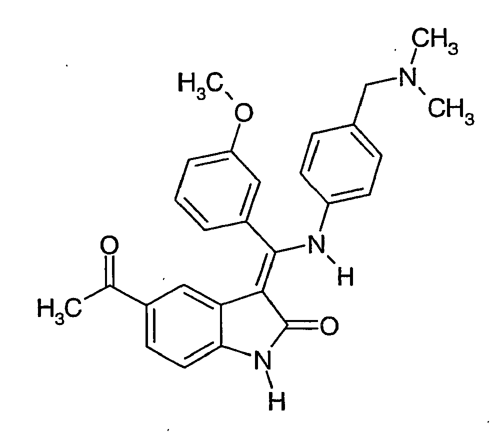
(d) 5-Acetyl-3-{[4-(dimethylaminomethyl)-phenylamino]-(3,5-dimethoxy-phenyl)-methyliden}-2-indolinon 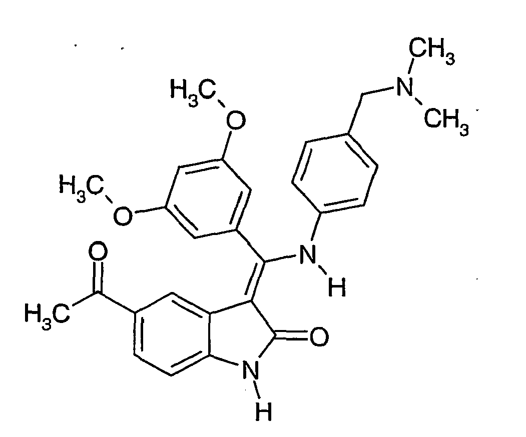
(e) 5-Acetyl-3-[(4-cyano-phenylamino)-(2,3-dihydro-benzo[1,4]dioxin-6-yl)-methyliden]-2-indolinon 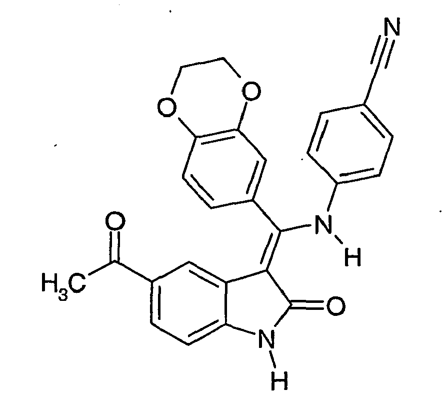
(f) 5-Acetyl-3-{[4-(ethylaminomethyl)-phenylamino]-phenyl-methyliden}-2-indolinon 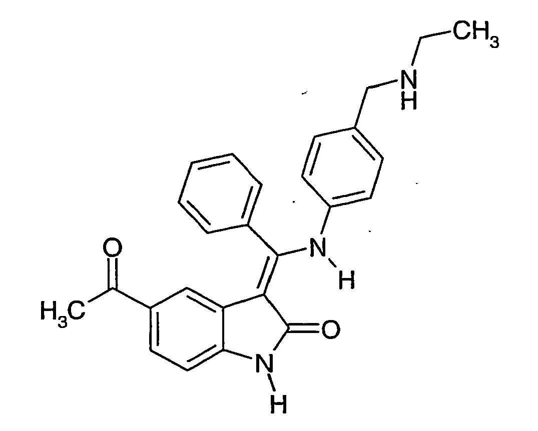
(g) 5-Acetyl-3-[1-(4-(dimethylaminomethyl)-phenylamino)-butyliden)-2-indolinon 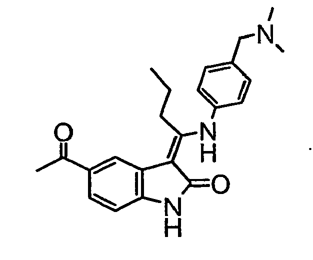
(h) 5-Acetyl-3-[1-(4-(dimethylaminomethyl)-phenylamino)-propyliden)-2-indolinon 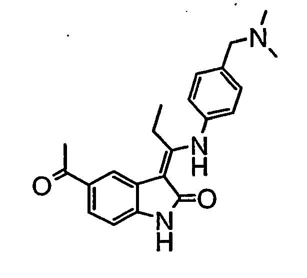
sowie deren Tautomere, Enantiomere, Diastereomere, deren Gemische und deren Salze.

7. Physiologisch verträgliche Salze der Verbindungen nach mindestens einem der Ansprüche 1 bis 6 mit anorganischen oder organischen Säuren oder Basen.

8. Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung nach mindestens einem der Ansprüche 1 bis 7 zur Herstellung eines Arzneimittels, das zur Behandlung von Diabetes mellitus Typ I und Typ II, Diabetes assoziierte Störungen wie diabetische Neuropathie, und degenerativer neurologischer Erkrankungen wie Alzheimers Erkrankung, Schlaganfall, neurotraumatische Verletzungen und bipolare Störungen geeignet ist.

10. Verfahren zur Herstellung eines Arzneimittels gemäß Anspruch 8, **dadurch gekennzeichnet, daß** auf nichtchemischen Weg eine Verbindung nach mindestens einem der Ansprüche 1 bis 7 in einen oder mehrere inerte Trägerstoffe und/oder Verdünnungsmittel eingearbeitet wird.

11. Verfahren zur Herstellung der Verbindungen der allgemeinen Formel I gemäß den Ansprüchen 1 bis 7, **dadurch gekennzeichnet, daß**
a) eine Verbindung der allgemeinen Formel 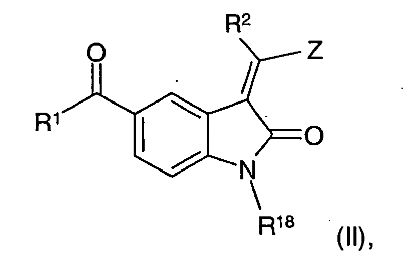 in der R¹ und R² wie in einem der Ansprüche 1 bis 6 erwähnt definiert sind,
R¹⁸ ein Wasserstoffatom oder eine Schutzgruppe für das Stickstoffatom der Lactamgruppe und
Z eine Austrittsgruppe darstellen,
mit einem Amin der allgemeinen Formel
R³-NH₂ (III)
,
in der R³ wie in einem der Ansprüche 1 bis 6 erwähnt definiert ist, wobei eventuell in den Resten R² und/oder R³ enthaltene Hydroxy-, Amino- oder Iminogruppen vorübergehend durch geeignete Schutzgruppen geschützt werden können, umgesetzt wird,
b) zur Herstellung einer Verbindung der Formel I, die eine Aminocarbonylgruppe enthält, eine Verbindung, die eine Carboxygruppe enthält, mit dem entsprechenden Amin umgesetzt wird,
c) zur Herstellung einer Verbindung der Formel I, die eine Carbonylaminogruppe enthält, eine Verbindung, die eine Aminogruppe enthält, mit dem entsprechenden Säurechlorid umgesetzt wird,
d) zur Herstellung einer Verbindung der Formel I, die eine Aminomethylgruppe enthält, eine Verbindung, die eine Cyanogruppe enthält, zu dem entsprechenden Aminomethylderivat hydriert wird,
e) zur Herstellung einer Verbindung der Formel I, die eine Aminogruppe enthält, eine Verbindung, die eine Nitrogruppe enthält, hydriert wird,
und/oder
anschließend gegegebenenfalls während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren oder Basen, übergeführt werden.

## Claims

1. Compounds of general formula 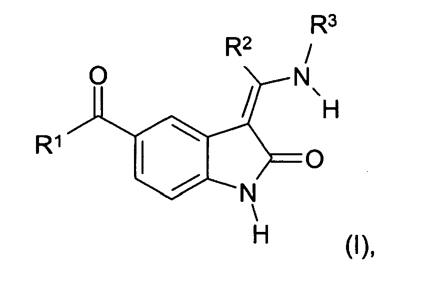 wherein
R¹ denotes a straight-chain or branched C₁₋₅-alkyl group wherein the hydrogen atoms may be wholly or partly replaced by fluorine atoms, or
an aryl group optionally substituted by a fluorine, chlorine or bromine atom,
while by an aryl group is meant a phenyl or naphthyl group,
R² denotes a C₁₋₇-alkyl or C₃₋₇-cycloalkyl group,
a 5- or 6-membered heteroaryl group with one to three heteroatoms selected from the group N, S and O, optionally substituted by one or two fluorine, chlorine, bromine or iodine atoms or one or two nitro, cyano, amino, C₁₋₃-alkyl or C₁₋₃-alkoxy groups, while both the heteratoms and the substituents may be identical or different,
a phenyl group wherein two adjacent carbon atoms are linked together through a methylenedioxy, ethylenedioxy or difluoromethylenedioxy group,
a phenyl group, to which another phenyl ring or a 5- or 6-membered heteroaromatic ring with one to three heteroatoms selected from the group N, S and O, while the heteratoms may be identical or different, is anellated, and while the bicyclic group may be substituted by one or two fluorine, chlorine, bromine or iodine atoms or one
or two nitro, cyano, amino, C₁₋₃-alkyl or C₁₋₃-alkoxy groups and the substituents may be identical or different, or
a phenyl group which may be substituted by one to three fluorine, chlorine, bromine or iodine atoms or by one to three C₁₋₃-alkyl, nitro, cyano, amino, di-(C₁₋₃-alkyl)-amino, C₁₋₃-alkyl-carbonylamino, phenylcarbonylamino, C₁₋₃-alkylsulphonylamino, arylsulphonylamino, trifluoromethyl, C₁₋₃ alkylsulphonyl, carboxy, C₁₋₃-alkoxy, di-(C₁₋₃-alkyl)-amino-C₁₋₃-alkyloxy, C₁₋₃-alkoxy-carbonyl, C₁₋₃-alkylaminocarbonyl, hydroxycarbonyl-C₁₋₃-alkyl-aminocarbonyl, C₁₋₃-alkoxycarbonyl-C₁₋₃-alkyl-aminocarbonyl, di-(C₁₋₃-alkyl)-amino-C₁₋₃-alkylaminocarbonyl, di-(C₁₋₃-alkyl)-amino-carbonyl-C₁₋₃-alkoxy, C₁₋₃-alkyl-amino-carbonyl-C₁₋₃-alkoxy, carboxy-C₁₋₃-alkoxy, C₁₋₃-alkyloxy-carbonyl-C₁₋₃ -alkoxy, carboxy-C₁₋₃-alkyl, C₁₋₃-alkoxy-carbonyl-C₁₋₃-alkyl, C₁₋₃-alkoxy-carbonylamino-C₁₋₃-alkyl, amino-C₁₋₃-alkyl, di-(C₁₋₃-alkyl)-amino-C₁₋₃-alkyl, C₁₋₃-alkyl-carbonylamino-C₁₋₃-alkyl, phthalimido, pyrrolyl or mono- or di-(C₁₋₃-alkyl)-pyrrolyl groups, while the substituents are identical or different, and
R³ denotes a phenyl, naphthyl or heteroaryl group as hereinbefore defined which may be mono-, di- or trisubstituted
by a fluorine, chlorine, bromine or iodine atom,
by a cyano, hydroxy, carboxy, C₁₋₃-alkoxy, C₁₋₃-alkoxycarbonyl or di-(C₁₋₃-alkyl)-amino-C₁₋₃-alkoxy group,
by a C₁₋₃-alkyl group which may be substituted by a hydroxycarbonyl, C₁₋₃-alkoxy-carbonyl or heteroaryl) group,
by a C₁₋₃-alkyl group which is substituted by a 3- to 7-membered cyclcoalkyleneimino group, while a benzene ring may be fused to the cycloalkyleneimino group via two adjacent carbon atoms,
by an amino-C₁₋₃-alkyl group which may be substituted at the nitrogen atom by one or two C₁₋₃-alkyl, phenyl-C₁₋₃-alkyl or C₁₋₄-alkoxy-carbonyl groups, while the substituents are identical or different,
by a C₁₋₃-alkyl-carbonyl-amino group which may be substituted at the nitrogen atom by a C₁₋₃-alkyl group or a C₂₋₃-alkyl group terminally substituted by a di-(C₁₋₃-alkyl)-amino group and in the alkyl moiety by a di-(C₁₋₃-alkyl)-amino, piperazinyl or 4-(C₁₋₃-alkyl)-piperazin-1-yl group,
by a C₂₋₃-alkyl-aminocarbonyl group terminally substituted in the alkyl moiety by a di-(C₁₋₃-alkyl)-amino group which may additionally be substituted at the nitrogen atom by a C₁₋₃-alkyl group,
or by a heteroaryl group,
while the substituents may be identical or different,
while the above-mentioned alkyl groups may be straight-chain or branched,
the tautomers, enantiomers, diastereomers, the mixtures thereof and the salts thereof.

2. Compounds of general formula I according to claim 1, wherein
R² and R³ are defined as in claim 1 and
R¹ denotes a methyl, ethyl, n-propyl, isopropyl, n-pentyl, trifluoromethyl or phenyl group,
the tautomers, enantiomers, diastereomers, the mixtures thereof and the salts thereof.

3. Compounds of general formula I according to claim 2, wherein
R¹ denotes a methyl, ethyl, n-propyl, isopropyl, n-pentyl or phenyl group,
R² denotes a C₁₋₇-alkyl group,
a phenyl group wherein two adjacent carbon atoms are linked together through a methylenedioxy, ethylenedioxy or difluoromethylenedioxy group, or
a phenyl group which may be substituted by one or two fluorine, chlorine, bromine or iodine atoms or by one or two C₁₋₃-alkyl, nitro, cyano, amino, C₁₋₃-alkylcarbonylamino, phenylcarbonylamino, C₁₋₃-alkylsulphonylamino, trifluoromethyl, carboxy, C₁₋₃-alkoxy, di-(C₁₋₃-alkyl)-amino-C₁₋₃-alkyloxy, C₁₋₃-alkoxy-carbonyl, C₁₋₃-alkylaminocarbonyl, hydroxycarbonyl-C₁₋₃-alkyl-aminocarbonyl, C₁₋₃-alkoxycarbonyl-C₁₋₃-alkylaminocarbonyl, di-(C₁₋₃-alkyl)-amino-C₁₋₃-alkylaminocarbonyl, carboxy-C₁₋₃-alkyl, C₁₋₃-alkoxy-carbonyl-C₁₋₃-alkyl, amino-C₁₋₃-alkyl or C₁₋₃-alkyl-carbonylamino-C₁₋₃-alkyl groups, while the substituents are identical or different, and
R³ denotes a phenyl group which may be mono- or disubstituted
by a fluorine, chlorine or bromine atom,
by a cyano, hydroxy, carboxy, C₁₋₃-alkoxy, C₁₋₃-alkoxycarbonyl or di-(C₁₋₃-alkyl)-amino-C₁₋₃-alkoxy group,
by a C₁₋₃-alkyl group which may be substituted by a hydroxycarbonyl, C₁₋₃-alkoxy-carbonyl or imidazolyl group,
by a C₁₋₃-alkyl group which is substituted by a 3- to 7-membered cyclcoalkyleneimino group, while a benzene ring may be fused to the cycloalkyleneimino group via two adjacent carbon atoms,
by an amino-C₁₋₃-alkyl group which may be substituted at the nitrogen atom by one or two C₁₋₃-alkyl, benzyl or C₁₋₄-alkoxy-carbonyl groups, while the substituents are identical or different,
by a C₁₋₃-alkyl-carbonyl-amino group which may be substituted at the nitrogen atom by a C₁₋₃-alkyl group or a C₂₋₃-alkyl group terminally substituted by a di-(C₁₋₃-alkyl)-amino group and in the alkyl moiety by a di-(C₁₋₃-alkyl)-amino, piperazinyl or 4-(C₁₋₃-alkyl)-piperazin-1-yl group,
by a C₂₋₃-alkyl-aminocarbonyl group terminally substituted in the alkyl moiety by a di-(C₁₋₃-alkyl)-amino group which may additionally be substituted at the nitrogen atom by a C₁₋₃-alkyl group,
or by an imidazolyl group
while the substituents may be identical or different,
the tautomers, enantiomers, diastereomers, the mixtures thereof and the salts thereof.

4. Compounds of general formula I according to claim 3, wherein
R¹ denotes a methyl group,
R² denotes an ethyl, propyl, butyl or pentyl group,
a phenyl group wherein two adjacent carbon atoms are linked together through a methylenedioxy, ethylenedioxy or difluoromethylenedioxy group, or
a phenyl group which may be substituted by one or two fluorine, chlorine, bromine atoms or by one or two C₁₋₃-alkyl, cyano, C₁₋₃-alkoxy, carboxy-C₁₋₃-alkyl, C₁₋₃-alkoxycarbonyl-C₁₋₃-alkyl, amino-C₁₋₃-alkyl or C₁₋₃-alkyl-carbonylamino-C₁₋₃-alkyl groups, while the substituents are identical or different, and
R³ denotes a phenyl group which may be monosubstituted
by a fluorine, chlorine or bromine atom,
by a cyano, carboxy, C₁₋₃-alkoxy or C₁₋₃-alkoxycarbonyl group,
by a C₁₋₃-alkyl group which may be substituted by a hydroxycarbonyl or C₁₋₃-alkoxy-carbonyl group,
by a C₁₋₃-alkyl group which is substituted by a 3- to 7-membered cyclcoalkyleneimino group,
by an amino-C₁₋₃-alkyl group which may be substituted at the nitrogen atom by one or two C₁₋₃-alkyl or C₁₋₄-alkoxy-carbonyl groups, while the substituents are identical or different,
by a C₁₋₃-alkyl-carbonyl-amino group which may be substituted at the nitrogen atom by a C₁₋₃-alkyl group or a C₂₋₃-alkyl group terminally substituted by a di-(C₁₋₃-alkyl)-amino group and in the alkyl moiety by a di-(C₁₋₃-alkyl)-amino or 4-(methyl)-piperazin-1-yl group,
or by a C₂₋₃-alkyl-aminocarbonyl group terminally substituted in the alkyl moiety by a di-(C₁₋₃-alkyl)-amino group which may additionally be substituted at the nitrogen atom by a C₁₋₃-alkyl group,
or may be disubstituted by a hydroxy and a di-(C₁₋₃-alkyl)-amino-C₁₋₃-alkyl group, while the substituents may be identical or different,
while the above-mentioned alkyl groups may be straight-chain or branched,
the tautomers, enantiomers, diastereomers, the mixtures thereof and the salts thereof.

5. Compounds of general formula I according to claim 4, wherein
R¹ denotes a methyl group,
R² denotes a phenyl group wherein two adjacent carbon atoms are linked together through a methylenedioxy or ethylenedioxy group, or
a phenyl group which may be substituted by one or two methoxy groups, and
R³ denotes a phenyl group which is substituted
by a cyano group or
by an amino-C₁₋₃-alkyl group which may be substituted at the nitrogen atom by one or two C₁₋₃-alkyl groups, while the substituents may be identical or different,
while the above-mentioned alkyl groups may be straight-chain or branched,
the tautomers, enantiomers, diastereomers, the mixtures thereof and the salts thereof.

6. The following compounds of general formula I according to claim 1:
(a) 5-acetyl-3-{[4-(diethylaminomethyl)-phenylamino]-phenyl-methylidene}-2-indolinone 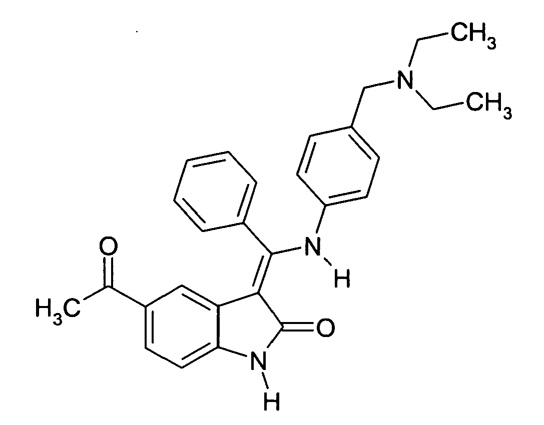
(b) 5-acetyl-3-{[4-(dimethylamino-methyl)-phenylamino]-(2,3-dihydro-benzo[1,4]-dioxin-6-yl)-methylidene}-2-indolinone 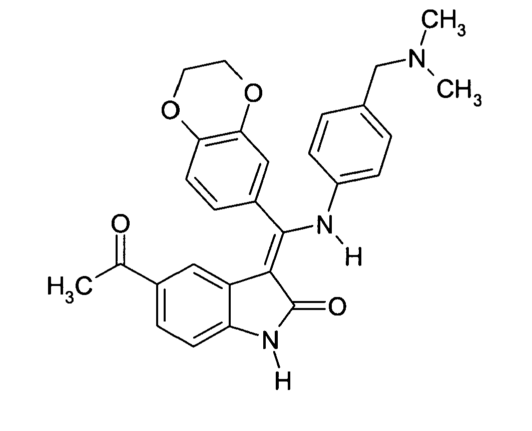
(c) 5-acetyl-3-{[4-(dimethylaminomethyl)-phenylamino]-(3-methoxy-phenyl)-methylidene}-2-indolinone 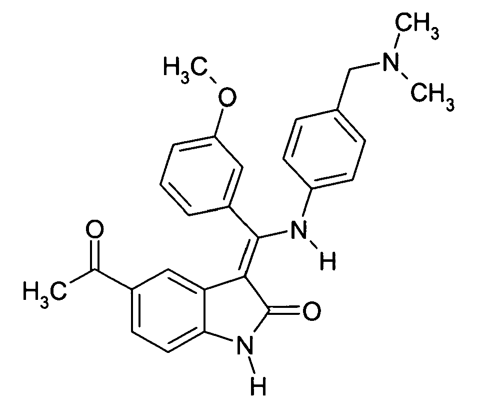
(d) 5-acetyl-3-{[4-(dimethylaminomethyl)-phenylamino]-(3,5-dimethoxy-phenyl)-methylidene}-2-indolinone 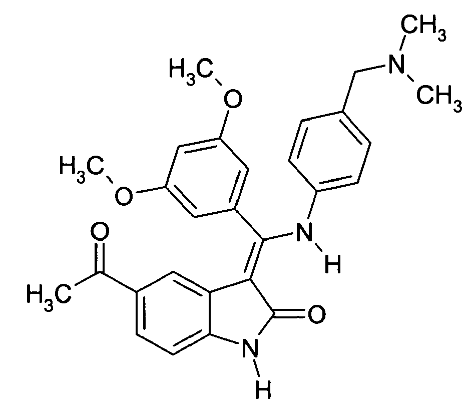
(e) 5-acetyl-3-[(4-cyano-phenylamino)-(2,3-dihydro-benzo[1,4]dioxin-6-yl)-methylidene]-2-indolinone 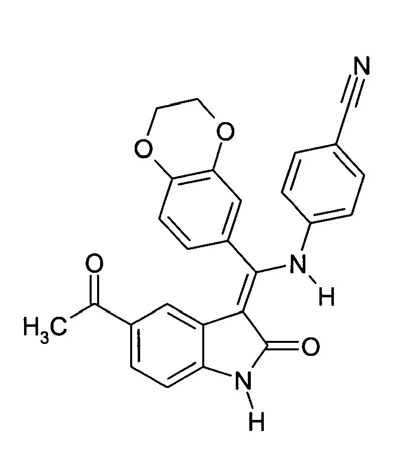
(f) 5-acetyl-3-{[4-(ethylaminomethyl)-phenylamino]-phenyl-methylidene}-2-indolinone 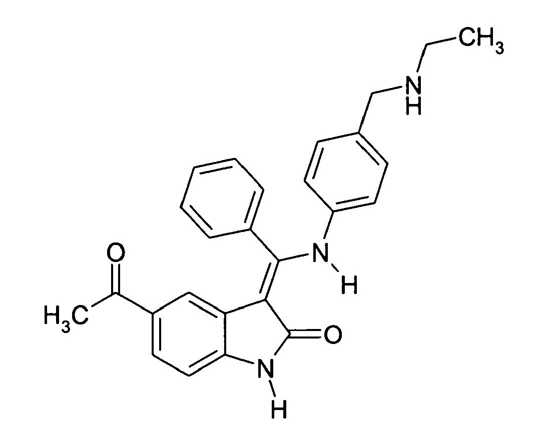
(g) 5-acetyl-3-[1-(4-(dimethylaminomethyl)-phenylamino)-butylidene)-2-indolinone 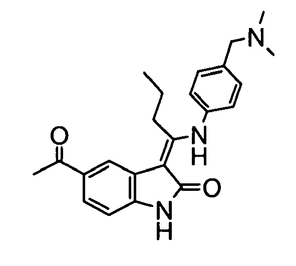
(h) 5-acetyl-3-[1-(4-(dimethylaminomethyl)-phenylamino)-propylidene)-2-indolinone 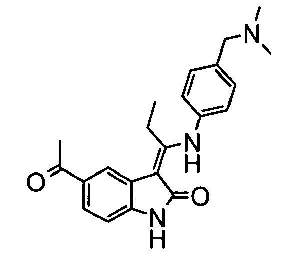 as well as the tautomers, enantiomers, diastereomers, the mixtures thereof and the salts thereof.

7. Physiologically acceptable salts of the compounds according to at least one of claims 1 to 6 with inorganic or organic acids or bases.

8. Pharmaceutical compositions containing a compound according to at least one of claims 1 to 6 or a physiologically acceptable salt according to claim 7 optionally together with one or more inert carriers and/or diluents.

9. Use of a compound according to at least one of claims 1 to 7 for preparing a pharmaceutical composition which is suitable for the treatment of type I and type II diabetes mellitus, diabetes associated disorders such as diabetic neuropathy and degenerative neurological diseases such as Alzheimer's disease, stroke, neurotraumatic injuries and bipolar disorders.

10. Process for preparing a pharmaceutical composition according to claim 8, **characterised in that** a compound according to at least one of claims 1 to 7 is incorporated in one or more inert carriers and/or diluents by a non-chemical method.

11. Process for preparing the compounds of general formula I according to claims 1 to 7, **characterised in that**
a) a compound of general formula 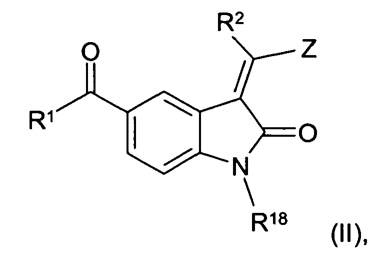 wherein R¹ and R² are defined as in one of claims 1 to 6,
R¹⁸ denotes a hydrogen atom or a protective group for the nitrogen atom of the lactam group and
Z denotes a leaving group,
is reacted with an amine of general formula
R³-NH₂ (III)
,
wherein R³ is defined as in one of claims 1 to 6, while any hydroxy, amino or imino groups contained in the groups R² and/or R³ may temporarily be protected by suitable protective groups,
b) in order to prepare a compound of formula I which contains an aminocarbonyl group, a compound which contains a carboxy group is reacted with the corresponding amine,
c) in order to prepare a compound of formula I which contains a carbonylamino group, a compound which contains an amino group is reacted with the corresponding acid chloride,
d) in order to prepare a compound of formula I which contains an aminomethyl group, a compound which contains a cyano group is hydrogenated to form the corresponding aminomethyl derivative,
e) in order to prepare a compound of formula I which contains an amino group, a compound which contains a nitro group is hydrogenated,
and/or
any protective groups which may be used during the reaction are then cleaved and/or
the compounds of general formula I thus obtained are resolved into their enantiomers and/or diastereomers and/or
the compounds of general formula I thus obtained are converted into their salts, particularly for pharmaceutical use into the physiologically acceptable salts thereof with inorganic or organic acids or bases.

## Revendications

1. Composés de formule générale 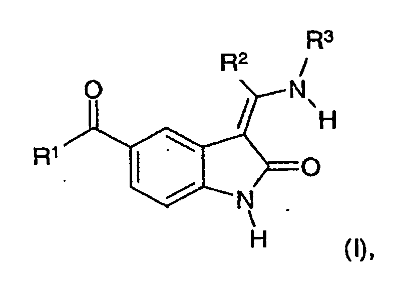 où
R¹ représente un groupe C₁₋₅-alkyle linéaire ou ramifié dans lequel les atomes d'hydrogène peuvent être remplacés totalement ou partiellement par des atomes de fluor, ou
un groupe aryle éventuellement substitué par un atome de fluor, de chlore ou de brome,
où par groupe aryle on doit entendre un groupe phényle ou naphtyle,
R² représente un groupe C₁₋₇-alkyle ou C₃₋₇-cycloalkyle,
un groupe hétéroaryle à 5 ou 6 chaînons éventuellement substitué par un ou deux atomes de fluor, de chlore, de brome ou d'iode ou un ou deux groupes nitro, cyano, amino, C₁₋₃-alkyle ou C₁₋₃-alcoxy avec un à trois hétéroatomes choisis dans le groupe N, S et O, où les hétéroatomes ainsi que les substituants peuvent être identiques ou différents,
un groupe phényle dans lequel deux atomes de carbone voisins sont liés entre eux par un groupe méthylènedioxy, éthylènedioxy ou difluorométhylènedioxy,
un groupe phényle auquel est condensé un autre groupe phényle ou un cycle hétéroaromatique à 5 ou 6 chaînons avec un à trois hétéroatomes choisis dans le groupe N, S et O, où les hétéroatomes peuvent être identiques ou différents, et où le bicycle peut être substitué par un ou deux atomes de fluor, de chlore, de brome ou d'iode ou un ou deux groupes nitro, cyano, amino, C₁₋₃-alkyle ou C₁₋₃-alcoxy et les substituants peuvent être identiques ou différents,
ou
un groupe phényle qui peut être substitué par un à trois atomes de fluor, de chlore, de brome ou d'iode ou par un à trois groupes C₁₋₃-alkyle, nitro, cyano, amino, di-(C₁₋₃-alkyl)amino, C₁₋₃-alkyl-carbonylamino, phénylcarbonylamino, C₁₋₃-alkylsulfonylamino, arylsulfonylamino, trifluorométhyle, C₁₋₃-alkylsulfonyle, carboxy, C₁₋₃-alcoxy, di-(C₁₋₃-alkyl)-amino-C₁₋₃-alkyloxy, C₁₋₃-alcoxy-carbonyle, C₁₋₃-alkylaminocarbonyle, hydroxycarbonyl-C₁₋₃-alkyl-aminocarbonyle, C₁₋₃-alcoxycarbonyl-C₁₋₃-alkyl-aminocarbonyle, di-(C₁₋₃-alkyl)-amino-C₁₋₃-alkylamino-carbonyle, di-(C₁₋₃-alkyl)-amino-carbonyl-C₁₋₃-alcoxy, C₁₋₃-alkyl-amino-carbonyl-C₁₋₃-alcoxy, carboxy-C₁₋₃-alcoxy, C₁₋₃-alkyloxy-carbonyl-C₁₋₃-alcoxy, carboxy-C₁₋₃ -alkyle, C₁₋₃-alcoxy-carbonyl-C₁₋₃-alkyle, C₁₋₃-alcoxy-carbonylamino-C₁₋₃-alkyle, amino-C₁₋₃-alkyle, di-(C₁₋₃-alkyl)-amino-C₁₋₃-alkyle, C₁₋₃-alkyl-carbonylamino-C₁₋₃ -alkyle, phtalimido, pyrrolyle ou mono- ou di-(C₁₋₃-alkyl)-pyrrolyle, où les substituants sont identiques ou différents, et
R³ représente un groupe phényle, naphtyle ou un groupe hétéroaryle défini comme ci-dessus qui peut être mono-, di- ou trisubstitué
par un atome de fluor, de chlore, de brome ou d'iode,
par un groupe cyano, hydroxy, carboxy, C₁₋₃-alcoxy, C₁₋₃-alcoxycarbonyle ou di-(C₁₋₃-alkyl)-amino-C₁₋₃-alcoxy,
par un groupe C₁₋₃-alkyle qui peut être substitué par un groupe hydroxycarbonyle, C₁₋₃-alcoxycarbonyle ou hétéroaryle,
par un groupe C₁₋₃-alkyle qui est substitué par un groupe cycloalkylèneimino à 3 à 7 chaînons où un cycle benzénique peut être condensé au groupe cycloalkylèneimino par le biais de deux atomes de carbone voisins,
par un groupe amino-C₁₋₃-alkyle qui peut être substitué sur l'atome d'azote par un ou deux groupes C₁₋₃-alkyle, phényl-C₁₋₃-alkyle ou C₁₋₄-alcoxy-carbonyle, où les substituants sont identiques ou différents,
par un groupe C₁₋₃-alkyl-carbonyl-amino qui peut être substitué sur l'atome d'azote par un groupe C₁₋₃-alkyle ou un groupe C₂₋₃-alkyle substitué en position terminale par un groupe di-(C₁₋₃-alkyl)-amino et dans la partie alkyle par un groupe di-(C₁₋₃-alkyl)-amino, pipérazinyle ou 4-(C₁₋₃-alkyl)-pipérazin-1-yle,
par un groupe C₂₋₃-alkyl-aminocarbonyle substitué dans la partie alkyle en position terminale par un groupe di-(C₁₋₃-alkyl)-amino, qui peut être substitué en outre sur l'atome d'azote par un groupe C₁₋₃-alkyle,
ou par un groupe hétéroaryle,
où les substituants peuvent être identiques ou différents,
où les groupes alkyle cités précédemment peuvent être linéaires ou ramifiés, leurs tautomères, leurs énantiomères, leurs diastéréoisomères, leurs mélanges et leurs sels.

2. Composés de formule générale I selon la revendication 1 dans lesquels
R² et R³ sont définis comme indiqué dans la revendication 1 et
R¹ représente un groupe méthyle, éthyle, n-propyle, isopropyle, n-pentyle, trifluorométhyle ou phényle,
leurs tautomères, leurs énantiomères, leurs diastéréoisomères, leurs mélanges et leurs sels.

3. Composés de formule générale I selon la revendication 2 dans lesquels
R¹ représente un groupe méthyle, éthyle, n-propyle, isopropyle, n-pentyle ou phényle,
R² représente un groupe C₁₋₇-alkyle,
un groupe phényle dans lequel deux atomes de carbone voisins sont liés entre eux par le biais d'un groupe méthylènedioxy, éthylènedioxy ou difluorométhylènedioxy,
ou
un groupe phényle qui peut être substitué par un ou deux atomes de fluor, de chlore, de brome ou d'iode ou par un ou deux groupes C₁₋₃-alkyle, nitro, cyano, amino, C₁₋₃-alkylcarbonylamino, phénylcarbonylamino, C₁₋₃-alkylsulfonylamino, trifluorométhyle, carboxy, C₁₋₃-alcoxy, di-(C₁₋₃-alkyl)-amino-C₁₋₃-alkyloxy, C₁₋₃-alcoxy-carbonyle, C₁₋₃-alkylaminocarbonyle, hydroxycarbonyl-C₁₋₃-alkyl-aminocarbonyle, C₁₋₃-alcoxycarbonyl-C₁₋₃-alkyl-aminocarbonyle, di-(C₁₋₃-alkyl)-amino-C₁₋₃-alkylaminocarbonyle, carboxy-C₁₋₃-alkyle, C₁₋₃-alcoxy-carbonyl-C₁₋₃-alkyle, amino-C₁₋₃-alkyle ou C₁₋₃-alkyl-carbonylamino-C₁₋₃-alkyle, où les substituants sont identiques ou différents, et
R³ représente un groupe phényle qui peut être mono- ou disubstitué
par un atome de fluor, de chlore ou de brome,
par un groupe cyano, hydroxy, carboxy, C₁₋₃-alcoxy, C₁₋₃-alcoxycarbonyle ou di-(C₁₋₃-alkyl)-amino-C₁₋₃-alcoxy,
par un groupe C₁₋₃-alkyle qui peut être substitué par un groupe hydroxycarbonyle, C₁₋₃-alcoxycarbonyle ou imidazolyle,
par un groupe C₁₋₃-alkyle qui est substitué par un groupe cycloalkylèneimino à 3 à 7 chaînons où un cycle benzénique peut être condensé au groupe cycloalkylèneimino par le biais de deux atomes de carbone voisins,
par un groupe amino-C₁₋₃-alkyle qui peut être substitué sur l'atome d'azote par un ou deux groupes C₁₋₃-alkyle, benzyle ou C₁₋₄-alcoxy-carbonyle, où les substituants sont identiques ou différents,
par un groupe C₁₋₃-alkyl-carbonyl-amino qui peut être substitué sur l'atome d'azote par un groupe C₁₋₃-alkyle ou un groupe C₂₋₃-alkyle substitué en position terminale par un groupe di-(C₁₋₃-alkyl)-amino et dans la partie alkyle par un groupe di-(C₁₋₃-alkyl)-amino, pipérazinyle ou 4-(C₁₋₃-alkyl)-pipérazin-1-yle,
par un groupe C₂₋₃-alkyl-aminocarbonyle substitué dans la partie alkyle en position terminale par un groupe di-(C₁₋₃-alkyl)-amino, qui peut être substitué en outre sur l'atome d'azote par un groupe C₁₋₃-alkyle,
ou par un groupe imidazolyle,
où les substituants peuvent être identiques ou différents,
leurs tautomères, leurs énantiomères, leurs diastéréoisomères, leurs mélanges et leurs sels.

4. Composés de formule générale I selon la revendication 3 dans lesquels
R¹ représente un groupe méthyle,
R² représente un groupe éthyle, propyle, butyle ou pentyle,
un groupe phényle dans lequel deux atomes de carbone voisins sont liés entre eux par le biais d'un groupe méthylènedioxy, éthylènedioxy ou difluorométhylènedioxy,
ou
un groupe phényle qui peut être substitué par un ou deux atomes de fluor, de chlore ou de brome ou par un ou deux groupes C₁₋₃-alkyle, cyano, C₁₋₃-alcoxy, carboxy-C₁₋₃-alkyle, C₁₋₃-alcoxy-carbonyl-C₁₋₃-alkyle, amino-C₁₋₃-alkyle ou C₁₋₃-alkyl-carbonylamino-C₁₋₃-alkyle, où les substituants sont identiques ou différents, et
R³ représente un groupe phényle qui peut être monosubstitué
par un atome de fluor, de chlore ou de brome,
par un groupe cyano, carboxy, C₁₋₃-alcoxy ou C₁₋₃-alcoxycarbonyle,
par un groupe C₁₋₃-alkyle qui peut être substitué par un groupe hydroxycarbonyle ou C₁₋₃-alcoxycarbonyle,
par un groupe C₁₋₃-alkyle qui est substitué par un groupe cycloalkylèneimino à 3 à 7 chaînons,
par un groupe amino-C₁₋₃-alkyle qui peut être substitué sur l'atome d'azote par un ou deux groupes C₁₋₃-alkyle ou C₁₋₄-alcoxy-carbonyle, où les substituants sont identiques ou différents,
par un groupe C₁₋₃-alkyl-carbonyl-amino qui peut être substitué sur l'atome d'azote par un groupe C₁₋₃-alkyle ou un groupe C₂₋₃-alkyle substitué en position terminale par un groupe di-(C₁₋₃-alkyl)-amino et dans la partie alkyle par un groupe di-(C₁₋₃-alkyl)-amino ou 4-(méthyl)-pipérazin-1-yle,
ou par un groupe C₂₋₃-alkyl-aminocarbonyle substitué dans la partie alkyle en position terminale par un groupe di-(C₁₋₃-alkyl)-amino, qui peut être substitué en outre sur l'atome d'azote par un groupe C₁₋₃-alkyle,
ou disubstitué par un groupe hydroxy et un groupe di-(C₁₋₃-alkyl)-amino-C₁₋₃-alkyle, où les substituants peuvent être identiques ou différents,
où les groupes alkyle cités précédemment peuvent être linéaires ou ramifiés, leurs tautomères, leurs énantiomères, leurs diastéréoisomères, leurs mélanges et leurs sels.

5. Composés de formule générale I selon la revendication 4 dans lesquels
R¹ représente un groupe méthyle,
R² représente un groupe phényle dans lequel deux atomes de carbone voisins sont liés entre eux par le biais d'un groupe méthylènedioxy ou éthylènedioxy, ou
un groupe phényle qui peut être substitué par un ou deux groupes méthoxy, et
R³ représente un groupe phényle qui est substitué
par un groupe cyano ou
par un groupe amino-C₁₋₃-alkyle qui peut être substitué sur l'atome d'azote par un ou deux groupes C₁₋₃-alkyle, où les substituants peuvent être identiques ou différents,
où les groupes alkyle cités précédemment peuvent être linéaires ou ramifiés, leurs tautomères, leurs énantiomères, leurs diastéréoisomères, leurs mélanges et leurs sels.

6. Composés de formule générale I selon la revendication 1 suivants :
(a) 5-acétyl-3-{[4-(diéthylaminométhyl)-phénylamino]-phényl-méthylidène}-2-indolinone 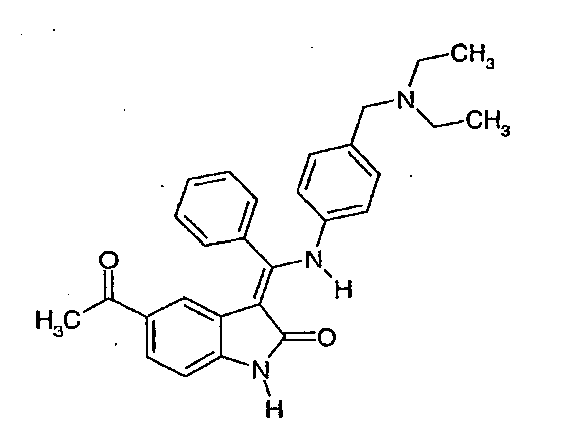
(b) 5-acétyl-3-{[4-(diméthylamino-méthyl)-phénylamino]-(2,3-dihydro-benzo-[1,4]dioxin-6-yl)-méthylidène}-2-indolinone 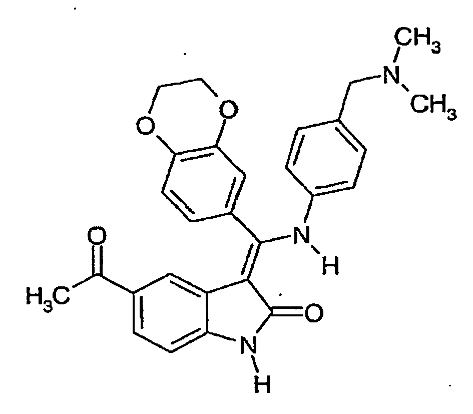
(c) 5-acétyl-3-{[4-(diméthylaminométhyl)-phénylamino]-(3-méthoxyphényl)-méthylidène}-2-indolinone 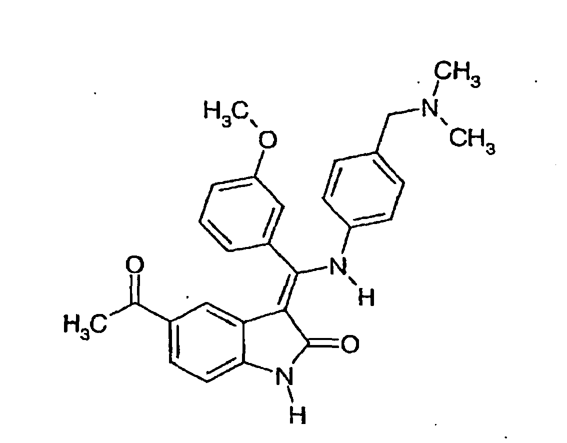
(d) 5-acétyl-3-{[4-(diméthylaminométhyl)-phénylamino]-(3,5-diméthoxyphényl)-méthylidène}-2-indolinone 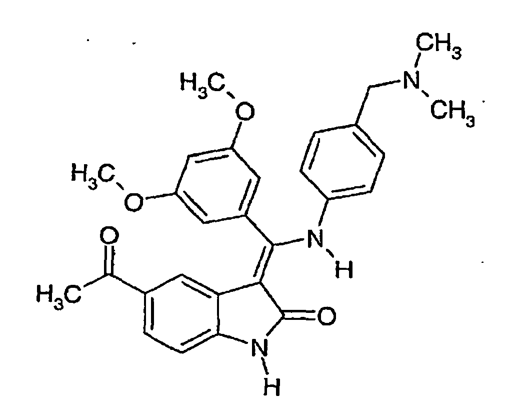
(e) 5-acétyl-3-[(4-cyano-phénylamino)-(2,3-dihydro-benzo[1,4]dioxin-6-yl)-méthylidène]-2-indolinone 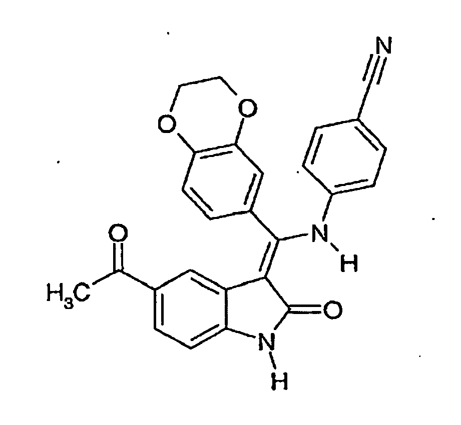
(f) 5-acétyl-3-{[4-(éthylaminométhyl)-phénylamino]-phényl-méthylidène}-2-indolinone 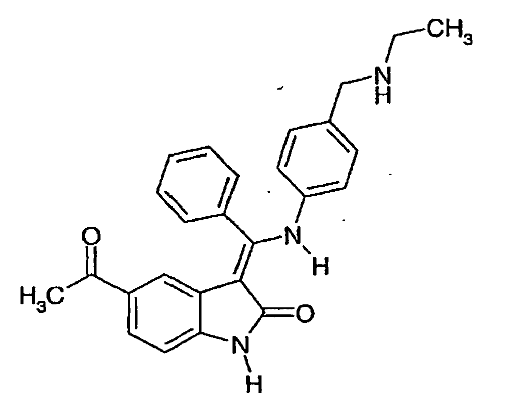
(g) 5-acétyl-3-[1-(4-(diméthylaminométhyl)-phénylamino)-butylidène)-2-indolinone 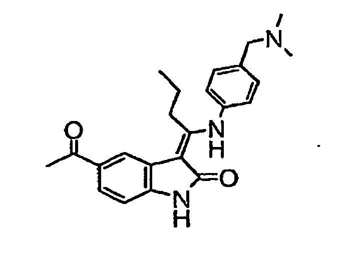
(h) 5-acétyl-3-[1-(4-(diméthylaminométhyl)-phénylamino)-propylidène)-2-indolinone 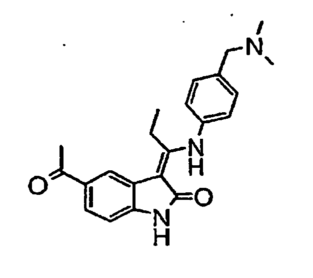 ainsi que leurs tautomères, leurs énantiomères, leurs diastéréoisomères, leurs mélanges et leurs sels.

7. Sels physiologiquement acceptables des composés selon au moins l'une des revendications 1 à 6 avec des acides ou bases inorganiques ou organiques.

8. Médicament contenant un composé selon au moins l'une des revendications 1 à 6 ou un sel physiologiquement acceptable selon la revendication 7 outre éventuellement un ou plusieurs vecteurs et/ou diluants inertes.

9. Utilisation d'un composé selon au moins l'une des revendications 1 à 7 pour la production d'un médicament qui convient pour le traitement du diabète sucré de type I et de type II, des troubles associés au diabète comme la neuropathie diabétique, et des maladies neurologiques dégénératives comme la maladie d'Alzheimer, l'apoplexie cérébrale, les lésions neurotraumatiques et les troubles bipolaires.

10. Procédé de production d'un médicament selon la revendication 8 **caractérisé en ce que**, par voie non chimique, un composé selon au moins l'une des revendications 1 à 7 est incorporé dans un ou plusieurs vecteurs et/ou diluants inertes.

11. Procédé de production des composés de formule générale I selon les revendications 1 à 7 **caractérisé en ce que**
a) un composé de formule générale 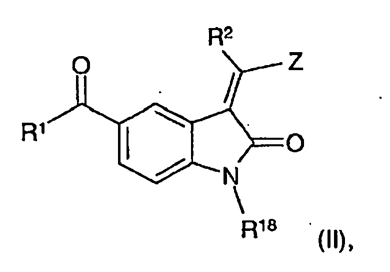 où R¹ et R² sont définis comme indiqué dans l'une des revendications 1 à 6,
R¹⁸ représente un atome d'hydrogène ou un groupe protecteur pour l'atome d'azote du groupe lactame et
Z représente un groupe partant,
est mis à réagir avec une amine de formule générale
R³-NH₂ (III)
où R³ est défini comme indiqué dans l'une des revendications 1 à 6, où les groupes hydroxy, amino ou imino éventuellement contenus dans les groupements R² et/ou R³ peuvent être protégés provisoirement par des groupes protecteurs appropriés,
b) pour la production d'un composé de formule I qui contient un groupe aminocarbonyle, un composé qui contient un groupe carboxy est mis à réagir avec l'amine correspondante,
c) pour la production d'un composé de formule I qui contient un groupe carbonylamino, un composé qui contient un groupe amino est mis à réagir avec le chlorure d'acide correspondant,
d) pour la production d'un composé de formule I qui contient un groupe aminométhyle, un composé qui contient un groupe cyano est hydrogéné en le dérivé aminométhylé correspondant,
e) pour la production d'un composé de formule I qui contient un groupe amino, un composé qui contient un groupe nitro est hydrogéné,
et/ou
ensuite les groupes protecteurs éventuellement utilisés pendant la réaction sont clivés et/ou
les composés de formule générale I ainsi obtenus sont séparés en leurs énantiomères et/ou diastéréoisomères et/ou
les composés de formule I obtenus sont convertis en leurs sels, en particulier pour l'utilisation pharmaceutique en leurs sels physiologiquement acceptables avec des acides ou bases inorganiques ou organiques.
